# EUROPÄISCHE PATENTSCHRIFT

(11) **EP 1 299 426 B1**
(45) Veröffentlichungstag und Bekanntmachung des Hinweises auf die Patenterteilung: **08.04.2009**
(21) Anmeldenummer: 01960439.6
(22) Anmeldetag: 02.07.2001
(51) Int. Cl.: C08F 2/24, G01N 33/545, C08F 2/44, C07B 57/00, B01D 69/12

(54) **VERBESSERTE MIKROGELE UND FILME**
IMPROVED MICROGELS AND FILMS
MICROGELS ET FILMS AMELIORES

(30) Priorität: 30.06.2000 DE 10031859
(43) Veröffentlichungstag der Anmeldung: 09.04.2003
(73) Patentinhaber: Fraunhofer-Gesellschaft zur Förderung der angewandten Forschung e.V., 80686 München (DE)
(72) Erfinder: TOVAR, Günter, D-70569 Stuttgart (DE); VAIHINGER, Dorothea, D-81549 München (DE); KRÄUTER, Iris, D-76185 Karlsruhe (DE); WEBER, Achim, D-73776 Altbach (DE); BRUNNER, Herwig, D-70569 Stuttgart (DE); HEROLD, Marc, D-70563 Stuttgart (DE)
(86) Internationale Anmeldenummer: PCT/EP2001/007525
(87) Internationale Veröffentlichungsnummer: WO 2002/002647

(56) Entgegenhaltungen:
- WO-A-95/03878
- US-A- 4 021 364
- US-A- 5 959 050
- MARIA J. UNZUÉ, ET.A.: "Reactive Surfactants in heterophase Polymerization." J. APPLIED POLYMER SCIENCE, Bd. 66, 1997, Seiten 1803-1820, XP002180422 in der Anmeldung erwähnt
- NATALIA PEREZ, MICHAEL J. WHITCOMBE, EVGENY N. VULSON: "Surface imprinting of cholesterol on submicrometer core-shell emulsion particles." MACROMOLECULES, Bd. 34, 2001, Seiten 830-836, XP002180423
- DATABASE WPI Section Ch, Week 200056 Derwent Publications Ltd., London, GB; Class A89, AN 2000-593239 XP002181559 & SE 9 802 911 A (HAUPT K), 29. Februar 2000 (2000-02-29)
- CRESCENZI V ET AL: "MOLECULARLY IMPRINTED POLYMERS BINDING CLENBUTEROL" , POLYMER PREPRINTS, AMERICAN CHEMICAL SOCIETY, US, VOL. 39, NR. 2, PAGE(S) 699-700 XP001031556 ISSN: 0032-3934 das ganze Dokument

## Beschreibung

Die vorliegende Erfindung betrifft verbesserte Mikrogele und daraus hergestellte Filme und Membranen sowie Verfahren zur Herstellung der Mikrogele, Filme und Membranen. Die erfindungsgemäßen Mikrogele, Filme und Membranen sind dadurch gekennzeichnet, dass sie Bereiche, zum Beispiel gebildet durch kovalent gebundene funktionelle Gruppen oder Kavitäten, zur molekülspezifischen Erkennung tragen. Die erfindungsgemäßen Mikrogele, Filme und Membranen können für chemo- oder/und biospezifische Prozesse der Separation, Sensorik, Analytik, Katalyse, Stoffproduktion, Medizintechnik oder ähnlichem eingesetzt werden.

Die Emulsionspolymerisation ist ein Spezialverfahren der Polymerisation, bei dem wasserunlösliche Monomere mit Hilfe von Emulgatoren in Wasser emulgiert und unter Verwendung wasserlöslicher Initiatoren, zum Beispiel Kaliumpersulfat, polymerisiert werden. Die bei der Emulsionspolymerisation anfallenden Polymerdispersionen, zum Beispiel Latex-Dispersionen, können für eine Vielzahl von Anwendungen eingesetzt werden. Die US 4,521,317 und US 4,021,364 zeigen Möglichkeiten und Grenzen der Emulsionspolymerisation auf.

Im Rahmen von Emulsionspolymerisationen können grundsätzlich zwei Typen von Emulsionen eingesetzt werden: die Öl-in-Wasser-Emulsion und die Wasser in Öl-Emulsion, auch inverse Emulsion genannt. In beiden Fällen wird ein im Reaktionsmedium unlösliches Monomer unter Rühren mittels Emulgatorzugabe dispergiert und die Reaktion durch Zugabe eines Initiators gestartet.

Die Emulsionspolymerisation lässt sich in drei Untertypen einteilen, deren Unterschiede hauptsächlich in der Größe der entstehenden Partikel bestehen: die Makroemulsion: Teilchengröße > 100 nm, die Miniemulsion: Teilchengröße 5 - 500 nm und die Mikroemulsion: Teilchengröße 5 - 50 nm.

Crescenzi et al. (Polymer Preprints, 1998, 39(2) 699-700) beschreiben die Herstellung von 15-25 µm großen Clenbuterol-bindenden Partikeln.

Die Herstellung von Biomosaikpolymeren in Form von Membranen, Filmen oder Partikeln mittels Emulsionspolymerisation wird in der EP-A 430 517 beschrieben. Die Polymerisation erfolgt im Wesentlichen in Gegenwart einer oberflächenaktiven Substanz (ionisches oder nicht ionisches Tensid) sowie einer biologisch aktiven Komponente (zum Beispiel Nucleinsäuren), die irreversibel in den Polymergrundkörper eingebaut beziehungsweise an dessen Oberfläche gebunden wird.

Ein Verfahren zur Herstellung biologisch aktiver polymerer Nanopartikel-Nucleinsäure-Konjungate ist in der WO 98/17317 beschrieben. Weitere Druckschriften, die den Stand der Technik sowie Möglichkeiten und Grenzen biofunktioneller Nanopartikel darstellen, sind die WO 96/20698, WO 96/05810 und Pérez et al. (Macromelecules, 2001, 34(4) 830-836).

Eine weitere Möglichkeit zur Synthese Oberflächenmodifizierter polymerer Nanopartikel stellt die Emulsionspolymerisation unter Einsatz von Surfmeren dar. Surfmere sind amphiphile Monomere (Surfmer = Surfactant + Monomer), die auf der Oberfläche von Latexpartikeln einpolymerisiert werden können und diese stabilisieren. Reaktive Surfmere verfügen zusätzlich über funktionalisierbare Endgruppen, die unter milden Bedingungen mit Nucleophilen, wie primären Aminen (Aminosäuren, Peptiden, Proteinen), Thiolen oder Alkoholen umgesetzt werden können. Auf diese Weise ist eine Vielzahl biologisch aktiver polymerer Nanopartikel zugänglich. Druckschriften, die den Stand der Technik sowie Möglichkeiten und Grenzen der Anwendung von Surfmeren wiedergeben, sind US 5, 177, 165, US 5, 525, 691, US 5, 162, 475, US 5, 827, 927, JP 10 018 092 und JP 4 018 929. Arbeiten zur Synthese von Surfmeren mit reaktiven Endgruppen wurden unter anderem von Nagai et al. (Polymer 1996, 37(1), 1257-1266; Journal of Colloid and Interface Science 1995, 172, 63-70), Unzué et al. (J. Applied Polym. Sci. 1997, 66, 1803-1820) und Guyot et al. (Curr. Opin. Colloid Interface Sci. 1996, 1(5), 580-586) veröffentlicht. Die Möglichkeit, funktionelle Surfmer-stabilisierte Latices zur Herstellung kohärenter Latexfilme einzusetzen, ist in den Schriften von Asua ausführlich diskutiert. Partikuläre Filme dieser Latices wurden in der Literatur noch nicht beschrieben.

Molekular geprägte Polymerfilme können gemäß der JP 11315150 durch ein neuartiges Gießverfahren hergestellt werden. Dabei wird eine wässrige Lösung aus Templat-bindendem Polymer und Templat mit einer organischen Polymerlösung gemischt, auf ein Substrat gegossen und das Lösungsmittel anschließend entfernt. Durch Waschen kann das Templat aus dem Film herausgelöst werden, so dass ein kohärenter Polymerfilm mit erkennenden Funktionen resultiert.

Ein in-situ-Verfahren zur Modifizierung von Mikrotiterplatten mit molekular geprägten Polymeren, welche in den einzelnen Wells der Mikrotiterplatte zu kohärenten, transparenten und kontinuierlichen Filmen verfestigt werden, ist in der DE 198 32 598 A1 beschrieben. Diese Mikrotiterplatten können zur molekularen Erkennung nach dem ELISA-Verfahren eingesetzt werden. Aufgrund des hohen Diffusionswiderstandes innerhalb des Films werden hier nur die Funktionen an der Oberfläche des Polymerfilms ausgenutzt.

Die Filmbildung von kohärenten Filmen ist ein bekannter Prozess, der ausführlich von Keddie (Mat. Sci. Eng., 1997, 21(3) 101-170) beschrieben wird. Dabei werden drei Stufen zur Bildung eines kohärenten Films aus einer wässrigen kolloidalen Dispersion von Polymerpartikeln beschrieben. Stufe 1: Verdunstung des Wassers und Ordnen der Partikel; Stufe 2: Partikeldeformation; Stufe 3: Interdiffusion von Polymeren über Partikel-Partikelgrenzen hinweg. Dabei erleidet der Latex folgende Umwandlungen: Kolloidale Dispersion → dichte Packung von Partikeln → dicht gepackte Ansammlung von deformierten Partikeln → kontinuierliches und undurchlässiges Material. Dieses Material ist wegen der Undurchlässigkeit per se ungeeignet zur Durchführung von kontinuierlichen Verfahren, zum Beispiel Filtration oder Osmose.

Database WPI Section (h, Week 200056 Derwent Publ. Ltd. (2000) beschreibt einen Film aus Polymeren, der durch Polymerisation in Gegenwart eines Porenerzeugenden Lösungsmittels hergestellt wird.

McDonald beschreibt in der WO 95/03878 die Darstellung von durchlässigen Verbundmaterialien aus unterschiedlich modifizierten Latices ausschließlich zur Größentrennung und damit Reinigung von Flüssigkeiten in der Mikro- und Ultrafiltrationstechnik. Die so hergestellten Membranen sind rissfrei, stabil gegenüber Durchfluss von Flüssigkeiten und Gasen sowie stabil gegenüber Rückfluss (back flushing). Funktionelle Gruppen in zusätzlich zugegebenen Monomeren, Surfactants, Oligomeren oder Polymeren - Glycidylmethylacrylat, Amid methylol, Methylmethacrylat, Glycidylmethylacrylat, Butylacrylat, Acrylsäure, Allylmethacrylat - werden ausschließlich zur Stabilisierung des Verbundmaterials verwendet. Die Ausnutzung der Kavitäten zum stoffabhängigen Transport oder Erkennungsprozessen in drei Dimensionen findet nicht statt und wird auch nicht als Möglichkeit erwähnt.

Die Herstellung molekular geprägter Polymerteilchen via Suspensions- und Bulkpolymerisation ist bekannt und zum Beispiel in den US 5,821,311, US 5,872,198, US 5,959,050 und WO 98/07671 beschrieben. Als "Molekulares Prägen" bezeichnet man eine Technik, die zur Darstellung von sehr hoch vernetzten Polymeren mit spezifischen molekularen Erkennungsfunktionen eingesetzt wird: Funktionelle Monomere werden in Gegenwart von Templatmolekülen, die durch Wechselwirkung mit dem Monomer eine Vorzugsausrichtung erfahren, polymerisiert. Durch Extraktion der Templatmoleküle erhält man ein molekular geprägtes Polymer, welches das Templat oder dem Templat strukturverwandte Molekülspezies durch Wechselwirkung mit den während des Prägeprozesses erzeugten Hohlräumen wieder selektiv binden kann und daher zur molekülspezifischen Erkennung geeignet ist.

Die Suspensionspolymerisation ermöglicht die kontrollierte Synthese einheitlicher, molekular geprägter Latexteilchen in einem Größenbereich von 2 µm - 100 µm. Aufgrund ihrer Größe sind solche Mikropartikel für medizinische in vivo Applikationen ungeeignet (DE 19 810 965 A1).

Bulkpolymerisate müssen vor ihrem Einsatz zum Beispiel als Füllmaterialien in Chromatographiesäulen mechanisch zerkleinert werden. Prinzipiell sind so gewonnene kleine Partikel völlig unregelmäßig in ihrer Größe und Morphologie. Durch Zerkleinerung gewonnene kleine polymere Partikel können nur verlustreich durch Siebprozess in ihrer Größe sortiert werden. Die Größe der auf diese Weise gewonnenen Partikel liegt typischerweise im Größenbereich von 5 - 500 µm.

Hosoya et al. stellten erstmals ein spezielles mehrstufiges Emulsionspolymerisationsverfahren zur Herstellung geprägter, quervernetzter Polymerbeads (≥ 5,5 µm) aus zum Beispiel Ethylendimethacrylat vor (J. High Resol. Chromatogr. 1999, 22 (5) 256 - 260; J. Chromatogr. A 1996, 728, 139-147; Chemistry Letters 1997, 1997(6), 555-556). Als Templatmolekül verwendeten sie u.a. Anthracen. Die erhaltenen Beads wurden als Füllmaterial in der HPLC eingesetzt und die Retentionszeiten von Benzol, Naphtalin und Anthracan untersucht. Für die chromatographische Anwendung sind Partikelgrößen von mindestens 3 µm erforderlich.

Eine Technik zur Oberflächen-Templat-Polymerisation unter Einsatz von Mikroemulsionen wurde von Uezu et al. entwickelt (Chemtech 1999, 29(4), 12-18; Journal of Chemical Engineering of Japan 1999 32 (3), 262-267; Journal of Applied Polymer Science 1999, 73 (7), 1223-1230). Es wird zuerst eine wässrige Lösung aus Templatmolekül und Emulgator in einer organischen Lösung aus Monomer und Templat-bindenden amphiphilen Surfactant emulgiert und anschließend das Monomer durch Zugabe eines Initiators polymerisiert. Die Templat-bindenden Moleküle lagern sich an der Grenzfläche Wasser/Öl an und erfahren durch die Bindung zum Templat eine Ausrichtung, die während beziehungsweise nach der Polymerisation erhalten bleibt. Durch Entfernung des Lösungsmittels und der Templatmoleküle erhält man ein poröses Polymer mit molekular geprägter, spezifischer innerer Oberfläche. Bei der konventionellen Emulsionspolymerisation findet die Polymerisation innerhalb von micellaren Öltröpfchen statt. Die Oberflächen-Templat-Polymerisation stellt somit ein inverses Verfahren zu einer Emulsionspolymerisation dar. Der Bereich der ursprünglichen Mikroemulsionen bleibt von der Polymerbildung ausgespart und wird nach Ausführung des Prozesses als Hohlraum abgebildet. Polymere lassen sich nach Uezu et al. dadurch einfach und mit zahlreichen wasserlöslichen Templatmolekülen und -atomen wie Aminosäuren, Proteinen und Metallionen prägen. Die Technik ist dadurch gekennzeichnet, dass die chemische Gruppen tragenden Moleküle, die für den Erkennungsprozess entscheidend sind, nicht kovalent mit dem Polymer verbunden sind. Nur in einem speziellen Fall berichtet Uezu von der Möglichkeit, derartige Moleküle nachträglich zum Präge- und Polymerisationsprozess durch Strahlung kovalent am Polymer zu verankern. Der Einsatz Oberflächen-Templat-geprägter Polymere zum Beispiel in Chromatographiesäulen erfordert eine mechanische Zerkleinerung des Materials auf die geeignete Korngröße. Die Bildung elastischer und mechanisch stabiler Polymerfilme ist mit diesem Verfahren nicht möglich.

Der Erfindung liegt also das technische Problem zugrunde, ein Verfahren zur Herstellung submikroskopischer Polymerpartikel mit molekülspezifisch erkennender Funktion und ein Verfahren zur Generierung von Filmen mit molekülspezifisch erkennender Funktion durch Verarbeitung submikroskopischer Polymerpartikel mit molekülspezifisch erkennender Funktion bereitzustellen, wobei die Funktion der Partikel an der Oberfläche der Filme und/oder im Inneren der Filme erhalten bleibt.

Die Erfindung löst die ihr zugrunde liegende technische Problemstellung durch die Bereitstellung molekülspezifische Erkennungsstellen aufweisender Nanopartikel mit einem Durchmesser von 20 bis 500 nm, die eine spezifische Wechselwirkung zwischen den Nanopartikeln und Zielmolekülen erlauben und die erhältlich sind durch

Herstellen einer Minimulsion, umfassend mindestens zwei nicht miteinander mischbare Phasen, enthaltend
a) mindestens ein Monomer,
b) mindestens ein Molekülspezifität verleihendes Agens, welches ein Templat oder ein oberflächenaktives polymerisierbares Monomer ist, wobei das oberflächenaktive polymerisierbare Monomer ein Vinyl-Monomer mit hydrophoben Segmenten und hydrophilen reaktiven Endgruppen ist und wobei die hydrophilen, reaktiven Endgruppen Sulfonium-aktivierte Ester- oder Succinimidester-Gruppen sind,
c) ein stabilisierendes Mittel, und
d) mindestens einen Emulgator und

Durchführen einer Miniemulsionspolymerisation und wobei die molekülspezifische Erkennungsstellen erhältlich sind durch Einpolymerisieren von Templaten und anschließendes Auswaschen oder durch Einpolymerisieren von oberflächenaktiven polymerisierbaren Monomeren.

In einer bevorzugten Ausführungsform ist vorgesehen, dass die Miniemulsion mindestens einen Initiator enthält.

In einer weiteren bevorzugten Ausführungsform ist vorgesehen, dass die Miniemulsion mindestens einen Vernetzer enthält.

In besonders bevorzugter Weise kann vorgesehen sein, die Emulsionspolymerisation durch thermisches oder photochemisches Starten einzuleiten. Es ist vorgesehen, der Emulsion vor Beginn der Polymerisation ein stabilisierendes Mittel in Form eines hydrophoben Agens hinzuzugeben.

Die Erfindung betrifft in einer besonders vorteilhaften Ausführungsform die vorgenannten Nanopartikel, die erhältlich sind durch
a) Herstellen eines Monomer-haltigen Gemisches aus mindestens einem Monomer sowie mindestens einem Molekülspezifität verleihenden Agens,
b) Herstellen einer Emulgator-haltigen Lösung,
c) Zugabe eines stabilisierenden Mittels zu dem Gemisch nach a) und/oder der Lösung nach b) und
d) Durchführen einer Miniemulsionspolymerisation durch Vereinigen der Emulgator-haltigen Lösung mit dem Monomer-haltigen Gemisch.

In einer bevorzugten Ausführungsform ist vorgesehen, dass in Schritt a) das Monomer-haltige Gemisch zusätzlich aus mindestens einem Initiator hergestellt wird.

In einer weiteren bevorzugten Ausführungsform ist vorgesehen, dass in Schritt a) das Monomer-haltige Gemisch zusätzlich aus mindestens einem Vernetzer hergestellt wird.

Die Erfindung sieht dabei in besonders vorteilhafter Weise vor, dass das die Molekülspezifität verleihende Agens beispielsweise ein Templat sein kann, wobei das zur Herstellung des Monomer-haltigen Gemisches eingesetzte Monomer mindestens eine funktionelle Gruppe aufweist, die zur Interaktion mit dem Templat befähigt ist. Nach Durchführen der Emulsionspolymerisation wird das Templat aus den gebildeten Nanopartikeln herausgewaschen, zum Beispiel herausgelöst beziehungsweise extrahiert.

Erfindungsgemäß kann jedoch auch vorgesehen sein, dass das die Molekülspezifität verleihende Agens ein oberflächenaktives polymerisierbares Monomer, auch als Surfmer bezeichnet, ist. Dieses oberflächenaktive polymerisierbare Monomer, insbesondere amphiphile Monomer, kann entweder selbst direkt molekülspezifische Erkennungsstellen tragen oder aber nach Durchführung mindestens einer, vorzugsweise milden, chemischen Reaktion molekülspezifische Erkennungsstellen aufweisen, die beispielsweise hinzugefügt oder demaskiert werden. Diese erfindungsgemäße chemische Reaktion kann sowohl vor als auch nach der Polymerisation erfolgen.

Die erfindungsgemäßen Nanopartikel werden durch die erfindungsgemäßen Verfahren hergestellt, die generell Verfahren der Emulsionspolymerisation zugeordnet werden können. Im Gegensatz zu herkömmlichen Verfahren der Suspensionspolymerisation werden dabei wesentlich kleinere Polymerpartikel erhalten.

Bei der Suspensionspolymerisation werden polymerisierbare Monomere in wenige Mikrometer große Tröpfchen in einem Dispersionsmittel verteilt und dann innerhalb dieser Tröpfchen durch Zugabe von in den Monomertröpfchen löslichen Initiatormolekülen und Starten der Reaktion polymerisiert. Die Reaktion läuft dabei innerhalb der Tröpfchen weitgehend so ab, wie bei einer Substanzpolymerisation (bulk polymerisation). Man verwendet daher für die Suspensionspolymerisation auch den Begriff der mikrobulk polymerisation. Das Verfahren der Suspensionspolymerisation ist in Bezug auf minimal erreichbare Polymerpartikelgrößen eingeschränkt. Minimale Partikelgrößen liegen bei wenigen Mikrometern.

Ohne durch die Theorie beschränkt sein zu wollen, erscheint es, als wenn durch die erfindungsgemäße Zusammensetzung der Emulsionen für die Polymerisation wesentlich kleinere Partikel erhalten werden, da die Polymerpartikel anders als in der Suspensionspolymerisation in den Monomertröpfchen in den in wesentlich größerer Zahl vorhandenen Emulgatorstabilisierten Mizellen heranwachsen und dafür die Diffusion der Monomere aus Monomertröpfchen in die Mizellen während des Polymerisationsprozesses eine entscheidende Rolle einnimmt.

Unter Emulgator wird im Zusammenhang mit der vorliegenden Erfindung ein Mittel verstanden, welches eine Oberflächenaktivität entwickelt, indem es die Grenzfläche zwischen den beiden nicht miteinander mischbaren Phasen stabilisiert. Der Emulgator kann vor Herstellen der Emulsion und Durchführung der Polymerisationsreaktion zugegeben werden und nicht in die entstehenden Polymere eingebunden werden oder kann eingebunden werden. Der Emulgator kann erfindungsgemäß selbstverständlich auch erst während der Durchführung der Polymerisation gebildet werden, zum Beispiel durch ein neues Molekül oder Ion gebildet aus dem Zerfallsprodukt des Initiatormoleküls und 1 bis 100, vorzugsweise 1 bis 20 Monomereinheiten.

Bei der erfindungsgemäßen Zugabe eines stabilisierenden Mittels zu der Emulsion, insbesondere bei der erfindungsgemäßen Miniemulsionspolymerisation, scheint es, dass das stabilisierende Mittel die vor der Polymerisation hergestellten Emulgatorstabilisierten Mizellen in ihrer Größenverteilung stabilisiert, indem durch den Aufbau eines osmotischen Druckes infolge der bevorzugten Löslichkeit des stabilisierenden Mittels in den Mizellen der Prozess der Ostwald-Reifung verhindert wird. Ohne Zugabe eines stabilisierenden Mittels scheint es, dass die erfindungsgemäß besonders bevorzugten Mizellengrößen und daraus resultierenden Partikelgrößen nicht stabil wären und durch den vor oder während der Polymerisation ablaufenden Prozess der Ostwald-Reifung eine wesentlich uneinheitlichere Partikelgrößenverteilung mit wesentlich weniger Partikeln in dem erfindungsgemäß besonders bevorzugtem Größenbereich entstehen würde.

Als stabilisierende Mittel werden erfindungsgemäß im Falle der hydrophobe-Phase-in-hydrophile-Phase-Emulsionen, wie Öl-in-Wasser-Emulsionen, hydrophobe Agentien verwendet. Erfindungsgemäß werden im Falle der hydrophile-Phase-in-hydrophobe-Phase-Emulsionen, wie Wasser-in-Öl-Emulsionen, hydrophile Agentien verwendet. Erfindungsgemäß können auch Template als stabilisierende Mittel wirken.

Unter Öl wird im Zusammenhang mit der vorliegenden Erfindung ein Mittel verstanden, welches eine organische Phase ist, die mit Wasser nicht oder nur zu einem geringen Teil, vorzugsweise zu weniger als 5 Gew.-% bezogen auf die Gesamtmenge des hier als Öl bezeichneten Mittels mischbar ist. Im Zusammenhang mit der vorliegenden Erfindung werden unter dem Begriff Wasser, reines Wasser ebenso wie wässrige Lösungen verstanden, die zum Beispiel Salze, Säuren, Basen, organische oder anorganische Verbindungen, Komplexe, tierische, pflanzliche, bakterielle Komponenten oder deren Bestandteile oder sonstige Inhaltsstoffe enthalten. Selbstverständlich können die vorgenannten möglicherweise im Wasser enthaltenden Komponenten auch in dem vorstehend definierten Öl vorhanden sein.

Im Zusammenhang mit der vorliegenden Erfindung werden unter dem Begriff Nanopartikel kolloidale Polymere verstanden, die hier auch als Latexpartikel bezeichnet werden. Diese Nanopartikel können in einer Ausführungsform als quervernetzte Nanopartikel vorliegen, die im vorliegenden Zusammenhang auch als quervernetzte Latexpartikel oder auch als Microgel bezeichnet werden. Oberflächenaktive polymerisierbare Monomere, das heißt Surfmere, enthaltende Nanopartikel sind nicht quervernetzt.

Die erfindungsgemäß hergestellten Nanopartikel mit einem Durchmesser von 5 nm bis 1 µm, vorzugsweise von 10 nm bis 0,8 µm, insbesondere von 20 nm bis 500 nm, liegen vorteilhafterweise in Form von kolloidalen Polymeren, also Latexpartikeln, insbesondere quervernetzten Latexpartikeln, also Microgelen, vor und ermöglichen in besonders vorteilhafter Weise die molekülspezifische Erkennung von Ziel-oder Targetmolekülen, wie Proteinen, pharmazeutischen Wirkstoffen, sonstigen Wert- oder Schadstoffen biotischen oder synthetischen Ursprungs, Bakterien, Viren, Viroiden, Prionen, Nucleinsäuren, Kofaktoren, Vitaminen, Aminosäuren, Kohlenhydraten, niedermolekularen organischen Verbindungen, höhermolekularen organischen Verbindungen und ähnlichem.

Erfindungsgemäß wird es ermöglicht, in einem Emulsionspolymerisationsverfahren, insbesondere einem Mini- oder Mikroemulsionspolymerisationsverfahren, in einem einstufigen Verfahren molekülspezifische Erkennungsstellen aufweisende polymere Nanopartikel in einem Größenbereich von 20 bis 500 nm zu synthetisieren. Erfindungsgemäß können die molekülspezifischen Erkennungsstellen durch Einpolymerisieren von Templaten während der Emulsionspolymerisation und anschließendes Auswaschen oder durch Einpolymerisieren von oberflächenaktiven polymerisierbaren Monomeren hergestellt werden. Erfindungsgemäß kann auch vorgesehen sein, die beiden vorgenannten Verfahren zur Einführung molekülspezifischer Erkennungsstellen zu kombinieren, das heißt in das Monomer-haltige Gemisch Template und oberflächenaktive polymerisierbare Monomere hinzuzugeben. Auf diese Weise werden molekülspezifische Erkennungsstellen in den erfindungsgemäßen Nanopartikeln sowohl durch die durch die herausgewaschenen Template gebildeten Kavitäten als auch durch die eingebauten oberflächenaktiven polymerisierbaren Monomeren bereitgestellt.

Erfindungsgemäß werden durch die erfindungsgemäße Vorgehensweise diskrete Partikel in kolloidaler Lösung erhalten, die über einen langen Zeitraum, vorzugsweise über sechs Monate, stabil ist. Die erhaltenen Polymere weisen eine partikuläre Struktur auf. Diese partikuläre Struktur ist weitgehend homogen, was einen weiteren Vorteil darstellt. So werden die herkömmlicherweise verwendeten Bulkpolymerisate für Anwendungen zum Beispiel in der Chromatographietechnik durch zeitaufwändige und kostspielige Verfahren mechanisch zerkleinert. Prinzipiell sind die so gewonnenen kleinen Partikel völlig unregelmäßig in ihrer Größe und Morphologie. Der Einsatz von Siebprozessen führt zu Verlusten. Die auf diese Weise gewonnenen Partikel weisen typischerweise Größen von 5 µm bis 500 µm auf. Die im Rahmen der konventionellen Suspensionspolymerisation gewonnenen molekülspezifischen Erkennungsstellen aufweisenden Polymerteilchen weisen Größen von 2 bis 100 µm auf. Erfindungsgemäß kann nun die Herstellung von molekulare Erkennungsstellen aufweisenden Partikeln im Größenbereich von 20 nm bis 500 nm realisiert werden.

Die durch das erfindungsgemäße Verfahren herstellbaren Partikel sind insbesondere dadurch gekennzeichnet, dass ihre Größe wesentlich geringer ist als die der durch Suspensionspolymerisation herstellbaren Partikel und die in ihrer Morphologie wesentlich einheitlicher sind, als alle durch herkömmliche mechanische Verfahren herstellbaren Partikel. Insbesondere werden die Grenzflächeneigenschaften der erfindungsgemäßen Partikel durch den Herstellungsprozess steuerbar. Die erfindungsgemäßen Partikel sind in kolloidaler Form leicht zu verarbeiten und für viele Anwendungen einsetzbar.

Im Zusammenhang mit der vorliegenden Erfindung werden unter molekülspezifischen Erkennungsstellen Bereiche des Nanopartikels verstanden, die eine spezifische Wechselwirkung zwischen dem Nanopartikel und Zielmolekülen, wie Liganden, ermöglichen. Die Wechselwirkung kann auf gerichteter attraktiver Wechselwirkung zwischen einem Paar, vorzugsweise mindestens zwei Paaren, aus funktionellen Gruppen des Nanopartikels und funktionellen Gruppen der Zielmoleküle beruhen. Einzelne interagierende Paare funktioneller Gruppen zwischen Nanopartikel und Zielmolekül sind jeweils räumlich fixiert an dem Nanopartikel und dem Zielmolekül angeordnet. Diese Fixierung braucht keine starre Anordnung zu sein, sondern kann vielmehr durchaus flexibel ausgeführt sein. Die attraktive Wechselwirkung zwischen den funktionellen Gruppen der Nanopartikel und der Zielmoleküle kann in Form von nicht-kovalenten Bindungen wie van-der-Waals-Bindungen, Wasserstoffbrücken-Bindungen, π-π-Bindungen, elektrostatischen Wechselwirkungen oder hydrophoben Wechselwirkungen ausgeführt sein. Denkbar sind auch reversible kovalente Bindungen ebenso wie Mechanismen, die auf Komplementarität der Gestalt oder Form beruhen. Die erfindungsgemäß vorgesehene Wechselwirkung zwischen den molekülspezifischen Erkennungsstellen der Nanopartikel und dem Zielmolekül beruhen also auf gerichteten Wechselwirkungen zwischen den Paaren aus funktionellen Gruppen und auf der räumlichen Anordnung dieser die Paarbildung eingehenden Gruppen zueinander an dem Nanopartikel sowie dem Zielmolekül.

Gemäß der vorliegenden Erfindung werden molekülspezifische Erkennungsstellen durch molekulares Prägen, den Einbau oberflächenaktiver polymerisierbarer Monomere oder beides bereitgestellt. Unter molekularem Prägen wird erfindungsgemäß die Polymerisation von Monomeren in Gegenwart von Templaten verstanden, die mit dem Monomer einen während der Polymerisation relativ stabilen Komplex bilden können. Nach dem Auswaschen der Template können die so hergestellten Materialien Templatmoleküle, den Templatmolekülen strukturverwandte Molekülspezies oder Moleküle, die den Templatmolekülen oder Teilen davon strukturverwandte oder identische Gruppen aufweisen, wieder spezifisch binden. Ein Templat ist daher eine in der Monomermischung während der Polymerisation vorhandene Substanz, zu der das gebildete Polymer eine Affinität aufweist.

Erfindungsgemäß kann jede Substanz mit definierter dreidimensionaler Gestalt oder Form als Templat für die Modifizierung von Nanopartikeln dienen. Erfindungsgemäß können sowohl kleine Moleküle mit Molekülmassen unter 100 Da als auch Partikel wie Viren, Bakterien, Zellen oder Zellorganellen als Templat dienen. Erfindungsgemäß können auch organische Verbindungen wie Aminosäuren, zum Beispiel Tryptophan oder Phenylalanin, Aminosäurederivate wie Bocphenylanalinanilid, Tryphtophanmethylester, Proteine, Lipide, Kohlenhydrate, Nucleinsäuren, Kofaktoren, Farbstoffe, Vitamine, Antikörper, Glycoproteine, Proteoglycane, Lektine, Monosaccharide, Disaccharide, Polysaccharide, Glycosaccharide oder heterozyklische Verbindungen als Templat verwendet werden.

Das Herauslösen des Templats nach dem Emulsionspolymerisationsvorgang kann beispielsweise durch Verwenden einer Salzlösung mit einer zur Dissoziation ausreichenden Ionenstärke geschehen. Selbstverständlich ist es auch möglich, Säuren einzusetzen, die die elektrostatischen Wechselwirkungen zwischen Templat und Polymerisat stören. Beim Herauslösen werden in den Poren und/oder an der Oberfläche der Nanopartikel die zur Templatstruktur komplementären Bindungsstellen freigesetzt.

Die Erfindung betrifft allerdings nicht nur Nanopartikel mit herausgelösten Templaten, sondern auch Nanopartikel, in denen oder an denen nicht herausgelöste Template noch, zumindest teilweise, gebunden sind.

Die Erfindung betrifft auch Nanopartikel, in die durch den Einbau oberflächenaktiver polymerisierbarer Monomere in Kombination mit molekularem Prägen oder ohne molekulares Prägen molekülspezifische Erkennungsstellen eingebracht wurden.

Im Zusammenhang mit der vorliegenden Erfindung werden unter oberflächenaktiven polymerisierbaren Monomere, also Surfmeren, amphiphile Monomere verstanden, die Vinyl-Monomere, zum Beispiel Methacryl-, Acryl-, Acrylamid-, Methacrylamid-, Maleinimid-, Citraconimid-, Itaconimid-, Styrol- oder Styrolsulfon-Monomere, mit hydrophoben Segmenten, zum Beispiel Methylen-, Ethylenoxid- oder Butylenoxid-Segmenten, und hydrophilen reaktiven Endgruppen wie Sulfonium-aktivierte Ester- oder Succinimidester-Gruppen sein können. Beispiele dafür sind Vinylalkyloyloxyphenyldimethylsulfoniumsalz, ω-Acrylamidoalkanoyloxyphenyldimethylsulfonium methylsulfat sowie Derivate dieser Verbindungen.

Die oberflächenaktiven polymerisierbaren Monomere weisen in einer Ausführungsform selbst molekülspezifische Erkennungsstellen auf, die Zielmoleküle erkennen und binden können. Selbstverständlich ist es auch möglich, dass die oberflächenaktiven polymerisierbaren Monomere nach Einpolymerisation erst durch eine nachfolgende sanfte chemische Reaktion mit molekülspezifischen Erkennungsstellen belegt werden. Dies kann durch Hinzufügen entsprechender Erkennungsstellen oder aber durch Demaskieren oder sonstiges Modifizieren geschehen.

Im Zusammenhang mit der vorliegenden Erfindung werden unter Zielmolekülen sämtliche Moleküle, Teile davon oder diese Moleküle tragenden Einheiten wie Zellen, Bakterien, Viren etc. verstanden, die von molekülspezifischen Erkennungsstellen erkannt und gebunden werden können. Beispiele für solche Zielmoleküle sind die vorgenannten Template.

Die Erfindung sieht vor, dass zur Herstellung eines Monomer-haltigen Gemisches mindestens ein Monomer mit mindestens einem Molekülspezifität verleihenden Agens, also einem Templat und/oder einem oberflächenaktiven polymerisierbaren Monomer vermischt wird. Erfindungsgemäß kann vorgesehen sein, dass das vorgenannte Monomer gleichzeitig auch das oberflächenaktive polymerisierbare Monomer darstellt. Es kann aber auch sein, dass verschiedene Monomere in dem Monomer-haltigen Gemisch vorliegen und demgemäß eine Copolymerisation durchgeführt wird.

Erfindungsgemäß kann das Monomer ein hydrophiles Monomer sein. Monomere mit hydrophilem Charakter werden in einer konventionell durchgeführten Öl-in-Wasser-Emulsionspolymerisation nicht oder nur in sehr geringer Rate in die gebildeten Polymere eingebaut, da das Polymerwachstum in der Ölphase stattfindet, hydrophile Monomere aber mit großem Verteilungsüberschuss in der Wasserphase gelöst sind. Überraschenderweise konnte durch die erfindungsgemäße Verfahrensweise ein Verfahren bereitgestellt werden, gemäß dem sowohl hydrophobe als auch hydrophile Monomere in das hergestellte Polymer durch Copolymerisation eingebunden werden.

Ohne durch die Theorie beschränkt sein zu wollen, erscheint es, als wenn durch die erfindungsgemäß besonders bevorzugte Zugabe eines stabilisierenden Mittels im Rahmen des erfindungsgemäßen Emulsionspolymerisationsverfahrens die Diffusion von hydrophilen Monomeren durch die wässrige Phase so eingeschränkt oder verhindert wird, dass eine erfolgreiche Polymerisation in der Ölphase stattfinden kann. Erfindungsgemäß können also sowohl hydrophobe als auch hydrophile Monomere in die Nanopartikel eingebaut werden.

In einer bevorzugten Ausführungsform der Erfindung ist das stabilisierende Mittel ein hydrophiles oder hydrophobes Agens.

In einer besonders bevorzugten Ausführungsform der Erfindung ist das hydrophobe Agens Hexadecan.

Erfindungsgemäß kann der eingesetzte Vernetzer ein hydrophober oder ein hydrophiler Vernetzer sein. Vernetzer können zum Beispiel Acrylatderivate wie Ethylenglycoldimethacrylat (EGDMA), N, N'-Methylenbisacrylamid, Trimethylolpropantrimethacrylat oder Bisphenol A oder Styrolderivate wie Divinylbenzol oder Diallylweinsäurediamid sein.

Ebenso kann der Initiator öl- oder wasserlöslich sein. Beispiele für geeignete Initiatoren sind Azoderivate, wie α,α'-Azoisobutyronitril, 2,2'-Azobis(2-methylbutyronitril), 1,1'-Azodi(hexahydrobenzonitrid), oder Peroxide wie Bis(α-α-dimethylbenzyl)peroxid oder Kaliumperoxodisulfat.

Als Monomer können acrylische Monomere, zum Beispiel Methacrylsäure, Acrylsäure, Acrylamid oder Methylmethacrylat, oder vinylische Monomere, zum Beispiel Vinylpyrrolidon, Vinylpyridin, Ethylstyrol, Vinylimidazol oder Itaconsäure verwendet werden.

Als Emulgator können ionische Emulgatoren, zum Beispiel Natriumdodecylsulfat oder Hexadecylsulfonsäure, kationische Emulgatoren, zum Beispiel Hexadecyltrimethylammoniumbromid oder Dodecylpyridiniumchlorid, oder nichtionische Emulgatoren, zum Beispiel die Brij-Reihe, insbesondere Brij 72^{®}, die Span-Reihe, insbesondere Span 20^{®} oder Span 80, oder die Tween-Reihe, insbesondere Tween 80^{®} verwendet werden. Der Emulgator wird vorzugsweise in Wasser beziehungsweise Cyclohexan im Falle der inversen Emulsion gelöst. Als Emulgator kann auch das Molekülspezifität verleihende Agens, insbesondere das oberflächenaktive polymerisierbare Monomer oder/und das Templat, verwendet werden.

Bezogen auf die Monomere setzt sich das Reaktionsgemisch bevorzugt wie folgt zusammen:
- Molares Verhältnis Vernetzer zu Monomer: 1:2 bis 1:4, falls Vernetzer vorhanden.
- Molares Verhältnis Monomer zu oberflächenaktivem Monomer: 1:5 bis 10:1, falls letzteres vorhanden.
- Molares Verhältnis Monomer zu Templat: 1:4 bis 1:10, falls letzteres vorhanden.
- 1 bis 5 Mol% stabilisierendes Mittel, falls vorhanden.
- 0,5 bis 2 Mol% Initiator, falls vorhanden.
- 0,2 bis 50 Mol% Emulgator.

Die erfindungsgemäßen Nanopartikel werden vorzugsweise in Form einer kolloidalen Dispersion, also von Latices, insbesondere quervernetzten Latices, also Mikrogele, erhalten.

Die erfindungsgemäßen Nanopartikel können auch in Form von Mikrogelen erhalten werden. Im Zusammenhang mit der vorliegenden Erfindung werden unter Mikrogelen durch den erfindungsgemäß bevorzugt vorgesehenen Einsatz von Vernetzern hergestellte quervernetzte Latices verstanden.

Die Erfindung betrifft auch Verfahren zur Herstellung molekülspezifische Erkennungsstellen aufweisender Nanopartikel der vorgenannten Art mit einem Durchmesser von 20 bis 500 nm, wobei die molekülspezifischen Erkennungsstellen eine spezifische Wechselwirkung zwischen dem Nanopartikel und Zielmolekülen erlauben und wobei eine mindestens zwei nicht miteinander mischbare Phasen, zum Beispiel eine hydrophile und eine hydrophobe Phase, umfassende Miniemulsion hergestellt wird, enthaltend
a) mindestens ein Monomer,
b) mindestens ein Templat oder mindestens ein oberflächenaktives polymerisierbares Monomer als ein Molekülspezifität verleihendes Agens, wobei das oberflächenaktive polymerisierbare Monomer ein Vinyl-Monomer mit hydrophoben Segmenten und hydrophilen reaktiven Endgruppen ist und wobei die hydrophilen, reaktiven Endgruppen Sulfonium-aktivierte Ester- oder Succinimid-ester-Gruppen sind,
c) ein stabilisierendes Mittel,
d) mindestens einen Emulgator,
und anschließend eine Miniemulsionspolymerisation durchgeführt wird, die in besonders bevorzugter Ausführungsform durch thermisches oder photochemisches Starten initiiert wird.

In einer bevorzugten Ausführungsform der Erfindung ist vorgesehen, dass die Miniemulsion mindestens einen Initiator enthält.

In einer weiteren bevorzugten Ausführungsform der Erfindung ist vorgesehen, dass die Miniemulsion mindestens einen Vernetzer enthält.

Erfindungsgemäß ist vorgesehen zur Durchführung einer Miniemulsion ein stabilisierendes Mittel, nämlich ein hydrophobes Agens, zum Beispiel Hexadecan, hinzuzugeben.

Die Erfindung betrifft in bevorzugter Ausführung auch vorgenannte Verfahren zur Herstellung molekülspezifische Erkennungsstellen aufweisender Nanopartikel der vorgenannten Art mit einem Durchmesser von 20 bis 500 nm, wobei ein Monomer-haltiges Gemisch aus mindestens einem Monomer, mindestens einem Molekülspezifität verleihenden Agens sowie eine Emulgator-haltige Lösung hergestellt werden, wobei das Monomer-haltige Gemisch und/oder die Emulgator-haltige Lösung mit einem stabilisierenden Mittel, zum Beispiel Hexadecan versetzt und wobei eine Miniemulsionspolymerisation durch Vereinigen der Emulgator-haltigen Lösung mit dem Monomer-haltigen Gemisch und anschließendem thermischen oder photoinitiierten Starten der Reaktion durchgeführt und eine die Nanopartikel enthaltende Suspension erhalten wird, welches anschließend gegebenenfalls durch zum Beispiel Filtration, insbesondere Diafiltration, so aufgereinigt werden kann, dass molekülspezifische Erkennungsstellen aufweisende Latices erhalten werden. Selbstverständlich ist es möglich, das stabilisierende Mittel nicht nur nach Herstellen des Monomer-haltigen Gemisches und/oder der Emulgator-haltigen Lösung hinzuzugeben, sondern auch bereits während der Herstellung.

In einer bevorzugten Ausführungsform der Erfindung ist vorgesehen, dass das Monomer-haltige Gemisch zusätzlich aus mindestens einem Initiator hergestellt wird.

In einer weiteren bevorzugten Ausführungsform der Erfindung ist vorgesehen, dass das Monomer-haltige Gemisch zusätzlich aus mindestens einem Vernetzer hergestellt wird.

Die Erfindung sieht also vor, dass eine bestimmte Menge an Emulgator in einer entsprechenden Menge Lösungsmittel, vorzugsweise Wasser oder Cyclohexan, gelöst wird. In einem getrennten Behältnis werden die Monomere mit einem Templat und/oder einem oberflächenaktiven polymerisierbaren Monomer sowie einem stabilisierenden Mittel und dem Initiator sowie gegebenenfalls dem Vernetzer gemischt. Beide Lösungen werden vereinigt, gründlich gerührt und gegebenenfalls mit einem Sonifier, der mit einer Sonotrode, vorzugsweise Mikrosonotrode, ausgestattet ist, beschallt.

Anschließend wird wahlweise thermisch oder photochemisch polymerisiert. Bei der thermischen Polymerisation wird die Temperatur auf 60 bis 100°C, insbesondere 80°C erhöht. Dieser Polymerisationsschritt kann beispielsweise 10 bis 20, insbesondere aber 16 Stunden, andauern. Anschließend wird die Temperatur erhöht, zum Beispiel um 5 bis 30°C, insbesondere um 10°C auf 90°C, und danach die Reaktion beendet. Bei der photoinitiierten Polymerisation wird das Reaktionsgefäß in einem Eisbad gekühlt, mit einer Standard UV-Lampe bestrahlt, vorzugsweise bei 366 nm, und 10 bis 20 Stunden, insbesondere aber 16 Stunden, polymerisiert und anschließend die Reaktion abgebrochen.

Eine Aufreinigung kann zum Beispiel über Ultrafiltration, Dialyse, Ausfällen der Mikrogele beziehungsweise Latices oder Ionenaustauscher erfolgen.

Die Erfindung betrifft in einer bevorzugten Ausführungsform eine Miniemulsionspolymerisation, die als hydrophobe-Phase-in-hydrophile-Phase-Miniemulsionspolymerisation, wie eine Öl-in-Wasser-Miniemulsionspolymerisation, ausgeführt wird. Die Erfindung betrifft selbstverständlich jedoch auch eine hydrophile-Phase-in-hydrophobe-Phase-Miniemulsionspolymerisation, wie Wasser-in-Öl-Miniemulsionspolymerisation, also eine inverse Miniemulsionspolymerisation.

In einer bevorzugten Ausführungsform der Erfindung ist das stabilisierende Mittel der hydrophobe-Phase-in-hydrophile-Phase-Miniemulsionspolymerisation ein hydrophobes Agens.

In einer bevorzugten Ausführungsform der Erfindung ist das stabilisierende Mittel der hydrophile-Phase-in-hydrophobe-Phase-Miniemulsionspolymerisation ein hydrophiles Agens.

In einer weiteren bevorzugten Ausführungsform ist die Durchführung einer Saatpolymerisation zu vorzugsweise Kern-Schale-Latices vorgesehen, wobei das Templat und/oder das oberflächenaktive polymerisierbare Monomer sowohl in der Saat beziehungsweise im Kern als auch in der Schale eingebaut sein können. Die Zugabe der einzelnen Komponenten, insbesondere der Monomere, erfolgt in bevorzugter Weise kontinuierlich. Im Zusammenhang mit der vorliegenden Erfindung werden Saatlatices mit Teilchengrößen von 20 bis 500 nm und Kern-Schale-Latices mit Teilchengrößen von 50 bis 500 nm erhalten.

Erfindungsgemäß ist vorgesehen, eine Miniemulsionspolymerisation durchzuführen. In einer besonders bevorzugten Ausführungsform ist vorgesehen, eine Mikroemulsionspolymerisation durchzuführen. Im Zusammenhang mit der vorliegenden Erfindung wird unter einer Miniemulsionspolymerisation eine Polymerisation verstanden, gemäß der Teilchengrößen von 20 bis 500 nm erhalten werden. Im Zusammenhang mit der vorliegenden Erfindung wird unter einer Mikroemulsionspolymerisation eine Polymerisation verstanden, gemäß der Teilchen von 5 bis 50 nm erhalten werden.

Wie bereits erläutert, sieht die Erfindung in einer Ausführungsform vor, dass das eingebaute Templat nach der Emulsionspolymerisation aus den gebildeten Nanopartikeln herausgelöst wird.

In einer weiteren bevorzugten Ausführungsform wird die Emulsionspolymerisation thermisch gestartet, insbesondere bei Temperaturen von 60 bis 100°C, insbesondere 70 bis 90°C, vorzugsweise 80°C. Vorzugsweise kann vorgesehen sein, die Temperatur stufenweise zu erhöhen. Die so hergestellten Nanopartikel sind aufgrund ihrer geringen Größe in besonders vorteilhafter Weise für diagnostische und therapeutische, zum Beispiel auch in vivo, Anwendungen geeignet, zum Beispiel, um mittels ihrer Molekülspezifität Zielstrukturen einbringen, neutralisieren, abfangen, detektieren oder zerstören zu können.

Darüber hinaus können die erfindungsgemäßen Nanopartikel katalytische Funktionen ausüben. Dies kann auch in in vivo-Anwendungen ausgenutzt werden.

Die so hergestellten Nanopartikel sind in kolloidaler Form leicht zu verarbeiten und für viele Anwendungen einsetzbar. So lassen sich durch Filtration partikuläre Nanoschichten, die ein- oder mehrschichtig sein können, herstellen. Diese Nanoschichten können aufgrund der starken Quervernetzung der in ihnen enthaltenen molekular geprägten Polymerteilchen mit einer Größe von 20 bis 500 nm zu partikulären Filmen verfestigt werden. Diese Filme oder Membranen weisen also eine partikuläre Grundstruktur insofern auf, als dass die sie bildenden Nanopartikel in ihnen im Wesentlichen in ihrer ursprünglichen Größe erhalten bleiben. Im Gegensatz zur Bildung kontinuierlicher Filme hat diese erfindungsgemäße Vorgehensweise den Vorteil, dass die spezifische Erkennung von Molekülen nicht nur an der Oberfläche des Polymerfilms abläuft, sondern auch die interpartikulären Zwischenräume im Film dazu ausgenutzt werden können. Erfindungsgemäß konnte die spezifische Erkennung von Molekülen innerhalb eines Polymerfilms daher erstmals von zwei auf drei Dimensionen ausgedehnt werden.

Die Erfindung betrifft daher auch Verfahren zur Herstellung eines Films oder einer Membran nach einem der vorhergehenden Verfahren, wobei mindestens ein Latex der vorgenannten Art mindestens einem Filtrationsschritt sowie einer vorzugsweise durchgeführten Trocknung ausgesetzt und ein partikulärer ein- oder mehrschichtiger Film oder Membran erhalten wird. In den so erhaltenen Filmen oder Membranen liegen interpartikuläre Zwischenräume vor, denen große Bedeutung insofern zukommt, als dass sie als Oberflächen mit molekülspezifisch erkennender Funktion im Film die Trennleistung der Membran entscheidend verbessern. Ein zu trennendes Stoffgemisch wird nicht nur ausschließlich aufgrund der Größe der Stoffe, sondern zusätzlich aufgrund seiner stofflichen Beschaffenheit durch die molekülspezifische Erkennung an der Partikeloberfläche und innerhalb des Partikels aufgetragen. Derartig hergestellte funktionelle polymere Kompositmembranen oder -filme können zum Beispiel in der Sensortechnik, in der Biomedizintechnik oder allgemein in der Separationstechnik, wie Filtration oder Osmose, Anwendung finden, insbesondere in der Flüssigchromatographie der kapillaren Elektrochromatographie oder der Festphasenextraktion.

Die Erfindung betrifft insbesondere die vorgenannten Filme oder Membrane, die eine Dicke von 10 nm bis 1000 µm, insbesondere 10 nm bis 500 µm aufweisen.

In einer weiteren bevorzugten Ausführungsform sind die Filme oder Membrane auf einem Substrat aufgebracht, welches zum Beispiel eine Sensoroberfläche, ein Stent, eine Filtermembran, ein Hohlfasermodul, ein Metall, ein Halbmetall wie Silizium, ein Metalloxid, Glas, Papier, ein Kunststoff wie Polyester, Polyarylsulfon, Polyethersulfon, Polyphenylsulfon, Polyvinylsulfon, Polystyrol, Polycarbonat, Polyethylen, Polypropylen, Polyacetylen, Polyvinylpiridin und Derivate, Polyvinylidenfluorid, Polyvinylchlorid, Polyvinylpyrolidon, Polytetrafluorethylen und Derivate, Nylon, Polyphenyloxid, verflochtene und verfilzte Fasern, mikroporöse Keramiken, Graphit, rostfreier Stahl, eine Membran, ein Filter, eine Folie oder ähnliches ist.

Die Erfindung betrifft auch einen vorgenannten Film oder Membran, wobei der Film oder die Membran mechanisch stabil und/oder permeabel und/oder regenerierbar ist. Im Zusammenhang mit der vorliegenden Erfindung wird unter regenerierbar die Eigenschaft eines Films, Membran oder auch eines Nanopartikels der Erfindung verstanden, gemäß der die molekularen Erkennungsstellen Zielmoleküle erkennen, binden und die Zielmoleküle auch wieder so abgelöst werden können, dass neue Zielmoleküle erkannt und gebunden werden können. Die Erfindung betrifft daher Filme, Membranen oder Nanopartikel, die sowohl reversibel als auch irreversibel mit ihren molekularen Erkennungsstellen Zielmoleküle binden.

Die Erfindung betrifft daher auch Verfahren zur Herstellung eines geträgerten erfindungsgemäßen Films oder Membran. In vorteilhafter Weise wird der Träger vor der Beschichtung mit dem Film oder der Membran vorbehandelt.

Die Figuren zeigen:
- Figur 1: zeigt die Topographie einer gemäß Bei- spiel 5 mit Mikrogel beschichteten Filte- rationsmembran/Kompositmembran.
- Figur 2: zeigt eine REM-Aufnahme (Rasterelektro- nenmikroskopie) eingetrockneter Partikel eines erfindungsgemäßen Mikrogels.
- Figur 3: zeigt die Ergebnisse mehrerer UV- spektrokopischer Untersuchungen.
- Figur 4: zeigt Isothermen der Titration von L-BFA geprägtem und ungeprägtem Mikrogel auf D- BFA und L-BFA.
- Figur 5: zeigt ¹H-NMR-Spektren von reinem L-BFA, L-BFA geprägtem Mikrogel und Mikrogel nach Templatextraktion.
- Figur 6: zeigt ein Massenspektrum der MALDI-TOF- MS-Messung von Latexpartikeln, an deren Oberfläche Streptavidin gebunden ist.

### Ausführungsbeispiel 1

72 mg des Emulgators SDS werden in 50 g Wasser unter Rühren gelöst. In einem zweiten Behälter werden 5,0 g Vernetzer (EGDMA) und 1,0 g Monomer (MAA) gemischt und 492 mg des Templats (Boc-Phenylalaninanilid, BFA) sowie 120 mg des Initiators (2,2'-Azobis-(2-methylbutyronitril)) und 250 mg Hexadecan darin gelöst. Die beiden Lösungen werden vereinigt und eine Stunde lang auf höchster Stufe des Magnetrührers gerührt. Anschließend wird die Emulsion zwei Minuten bei 50% Amplitude in einer Sonifier-Vorrichtung beschallt. Die Emulsion wird hierbei in ein Eisbad gestellt. Sofort im Anschluss wird 16 h bei 80°C polymerisiert. Gegen Ende der Reaktion wird die Ölbadtemperatur für eine Stunde auf 90°C erhöht, um einen möglichst hohen Umsatz zu gewährleisten.

Nach Beendigung der Reaktion wird das gebildete Koagulat durch Filtration abgetrennt und der Feststoffgehalt der Probe bestimmt. Die Teilchengröße wird mittels einer Zetasizer 3000HS-Vorrichtung der Firma Malvern bestimmt. Sie liegt für geprägte Latices dieser Zusammensetzung zwischen 180 und 195 nm.

### Ausführungsbeispiel 2

In 50 g Wasser werden 72 mg Natriumdodecylsulfat gelöst. In einem zweiten Behältnis mischt man 5,0 g Divinylbenzol mit 1,0 g Methacrylsäure und löst darin 120 mg des Initiators 2,2'-Azobis(2-methylbutyronitril), 250 mg Hexadecan und 200 mg Templat. Die beiden Lösungen werden vereinigt und eine Stunde lang auf höchster Stufe des Magnetrührers gerührt. Anschließend wird die Emulsion zwei Minuten bei 50% Amplitude in einer Sonifier-Vorrichtung beschallt. Die Emulsion wird hierbei in ein Eisbad gestellt. Anschließend erfolgt die thermisch iniziierte Polymerisation bei 80°C über 16 h. Gegen Ende der Reaktion wird die Ölbadtemperatur für eine Stunde auf 90°C erhöht, um einen möglichst hohen Umsatz zu gewährleisten.

Die mittlere Partikelgröße liegt bei 220 nm.

### Ausführungsbeispiel 3

600 mg Span 80^{®} werden in 60 g Cyclohexan unter Rühren gelöst. In 5 g Wasser löst man 250 mg N,N'-Methylenbisacrylamid und 65 mg Acrylamid sowie 35 mg Mandelsäure und 7 mg Kaliumperoxodisulfat. Beide Lösungen werden vereinigt und 1 h auf höchster Stufe des Magnetrührers gerührt. Anschließend wird die Probe 2 min bei 50% Amplitude in einer Sonifier-Vorrichtung gestellt, die mit einer Mikrosonotrode ausgestattet ist. Dann wird 5 h bei 70°C und eine weitere Stunde bei 80°C polymerisiert.

### Ausführungsbeispiel 4

In 45 g Cyclohexan werden 460 mg Span 80^{®} gelöst. In einem zweiten Behältnis werden 500 mg Diaminweinsäurediallylamid, 125 mg Acrylamid, 72 mg Mandelsäure und 7 mg Kaliumperoxodisulfat in 3 g Wasser gelöst. Nach Vereinigung beider Lösungen wird eine Stunde auf höchster Stufe des Magnetrührers gerührt und daraufhin 2 Minuten bei 50% Amplitude in einer Sonifier-Vorrichtung beschallt. Polymerisiert wird für 16 h bei 70°C.

### Ausführungsbeispiel 5

Eine geeignete Filtrationsmembran wird als Träger gewählt und vor der Beschichtung 15 Minuten in Wasser gelegt. Die Beschichtung erfolgt in einer Amicon-Filtrationszelle, in welche die Membran eingespannt wird. Ein bestimmtes Volumen einer Latex-Suspension mit einem definierten Gehalt an kolloidalem Latex wird in die Zelle eingefüllt und mit leichtem Überdruck durchfiltriert. Ober die Konzentration der Suspension oder deren Volumen kann die gewünschte Schichtdicke eingestellt werden.

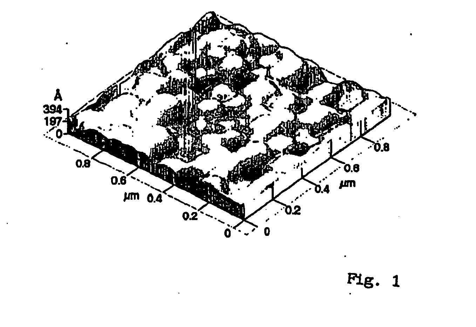

Die beschichteten Membranen werden zunächst bei 40°C und 70% relativer Luftfeuchtigkeit drei Stunden getrocknet und anschließend noch eine Stunde bei 120°C. Figur 1 zeigt eine wie vorstehend beschichtete Filtrationsmembran. Deutlich zu erkennen ist die partikuläre Struktur des aufgebrachten Mikrogels, also der Oberfläche der Kompositmembran. Die Aufnahme erstand mittels Atomarer Kraftmikroskopie (Rasterkraftmikroskopie, AFM).

### Ausführungsbeispiel 6

Mittels einer Miniemulsionspolymerisation wurden molekular geprägte Mikrosphären beziehungsweise Nanopartikel und Mikrogele hergestellt.

5,4 g (27,25 mmol) Vernetzer (EGDMA; Sigma (vor Gebrauch mit 10 % NaOH gewaschen, über MgSO₄ getrocknet und destilliert) oder DVB, Divinylbenzol), 0,6 g (6,9 mmol) funktionales Monomer (Methacrylsäure, MAA), 250 mg Hexadecan als hydrophobes Agens und 120 mg 2,2'-Azobis (2-Methylbutyronitril) wurden gemischt. Die mittels Miniemulsionspolymerisation hergestellten vernetzten Mikrogele wurden mit und ohne Templat hergestellt. Im Falle des molekularen Prägens wurde ein Enantiomeren-reines L- oder D-Boc-Phenylalaninanilid (L-oder D-BFA) (495 mg, 1,4 mmol) zugegeben. 72 mg Natriumdodecylsulfat SDS, als oberflächenaktives Agens, wurden in 50 g Wasser gelöst. Die Lösungen wurden gemischt, eine Stunde kräftig gerührt und anschließend 2 Minuten beschallt (Branson Digital Sonifier, Modell 450-D, 60 % Leistung). Die Polymerisation wurde thermisch bei 80°C für 16 Stunden initiiert. Sich gegebenenfalls bildende Coagulate wurden durch Filtration entfernt. Die Mikrogele wurden anschließend mittels Diafiltration (Amicon 8400 Ultrafiltrationszelle, Membran Polysulfon) gereinigt. Unter Verwendung unterschiedlicher Vernetzermengen wurden unterschiedliche Verhältnisse von Monomer zu Vernetzer eingesetzt.

Das molare Verhältnis des funktionalen Monomers zu dem Vernetzer n_{MAA}/n_{CL} betrug 1:2,2, 1:4 oder 0 im Falle von EGDMA und 1:3,3 oder 0 im Fall von DVB (Divinylbenzol) als Vernetzer.

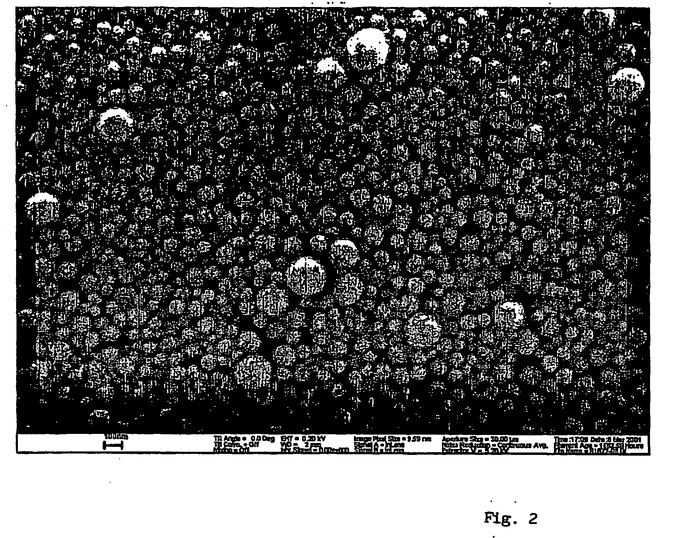

Die Miniemulsionen wurden in Mikrogele mit einer Effizienz zwischen 90 und 95 % konvertiert. Die erhaltenen erfindungsgemäßen Mikrogele wurden mittels Dynamic Light Scattering (Dynamische Lichtstreuung), gravimetrischer Analysen, Oberflächenspannungstitration, Gasabsorptionsmessungen und Rasterelektronenmikroskopie charakterisiert. Figur 2 zeigt ein wie vorstehend erhaltenes Mikrogel mit seiner partikulären Struktur, wobei zu erkennen ist, dass die Mikrogele Durchmesser von 50 - 250 mm aufweisen und die Verteilung der sphärischen Partikel diskret ist.

Die durchschnittlichen Partikeldurchmesser betrugen 200 ± 25 nm (gemessen mit dynamischer Lichtstreuung, diese Meßmethode wurde für alle im folgenden aufgeführten Durchmessermessungen eingesetzt). Der Durchmesser der molekular geprägten Mikrogele umfassend MAA-co-EGDMA betrugen 217 nm, 204 nm, 220 nm und 215 nm. Die entsprechenden nicht molekular geprägten Mikrogele zeigten eine größere Variation der Durchmesser mit 187 nm, 255 nm und 209 nm. Die DVB enthaltenen Mikrogele wiesen Durchmesser von 222 nm, 188 nm und 222 nm auf. Die Größenvarianz σ² aller Mikrogele betrug σ² = 0,12 bis 0,14.

Die Oberflächenspannung σ wurde nach Reaktionsabschluss an der Luft-Wasser-Grenzfläche gemessen. Alle Miniemulsionen enthielten dieselbe Menge an SDS und die Oberflächenspannung zeigt daher direkt die Menge freien SDS in Lösung an. Je höher die gemessene Oberflächenspannung σ ist, desto geringer ist der Gehalt an SDS in Lösung und umso mehr SDS muss von den gebildeten Mikrogelen adsorbiert worden sein. Die Oberflächenspannung σ wurde für das System MAA-co-EGDMA als konstant ermittelt, unabhängig von dem molaren Verhältnis oder der Abwesenheit oder Anwesenheit des Templats mit σ = 43,4 ± 1,2 mN m⁻¹. Das reine EGDMA-Mikrogelreaktionsgemisch zeigt eine beträchtlich höhere Oberflächenspannung nach erfolgter Polymerisation mit σ = 62,1 mN m⁻¹. Die Poly(MAA)-co-(DVB) Reaktionsgemische zeigten eine Oberflächenspannung σ von 59,6 mN m⁻¹ oder 55,6 mN m¹ in der Gegenwart oder Abwesenheit des Templats. Polymerisation reinen DVBs führte zu einer Oberflächenspannung von σ = 64,4 mN m⁻¹. Dieser Wert befindet sich in derselben Größenordnung wie für reine EGDMA-Mikrogele. Alle MAA enthaltenden Miniemulsionsreaktionsgemische zeigten eine erheblich geringere Oberflächenspannung als reine Vernetzungssysteme.

Die Topologie der Mikrogele im trockenen Zustand wurde mittels Stickstoffadsorptions- und gravimetrischer Analysen bestimmt. Die gemessenen spezifischen Oberflächen σ_{A} der gereinigten und getrockneten Mikrogele variierten mit der chemischen Zusammensetzung der Mikrogele. Poly(MAA)-co-(EGDMA)Mikrogele, die in einem molaren Verhältnis von 1:2 funktionales Monomer zu Vernetzer hergestellt wurden, resultierten in einem σ_{A} = 57,1 m² g⁻¹ in Gegenwart des Templats und σ_{A} = 53,2 m² g⁻¹ in Abwesenheit eines Templats. Bei Änderung des molaren Verhältnisses zu 1:4 veränderte sich die spezifische Oberfläche zu σ_{A} = 64,9 m² g⁻¹. Auch bei den aus MAA-DVB hergestellten Partikeln war die spezifische Oberfläche in Gegenwart eines Templats größer, und zwar betrug σ_{A} = 70,0 m² g⁻¹ in Anwesenheit des Templats und σ_{A} = 61,3 m² g⁻¹ ohne Templat.

### Ausführungsbeispiel 7

### Templatbindung

In Anwesenheit von Templat synthetisierte Mikrogele wurden vor den Bindeexperimenten extrahiert. Die Extraktionseffizienz kann durch Kalkulation wie folgt ermittelt werden: Aus der Zusammensetzung der Ausgangsmaterialien ist bekannt, dass in 400 mg Mikrogel maximal 83,9 µmol BFA enthalten sein können, wobei mögliche Verluste des Templats während der Herstellung und Reinigung der Mikrogele nicht berücksichtigt werden. Der erste Extraktionsschritt entfernte bereits 49,3 µmol BFA aus dem Mikrogel, was 59 % entspricht. Durch viermalige Wiederholung des Extraktionsschrittes konnten 64,5 µmol BFA extrahiert werden, was 77 % entspricht. Mit D-BFA anstelle von L-BFA hergestellte Mikrogele erlauben eine Extraktion bis zu 72 %.

Für die Bindeexperimente mit Templaten wurden 400 mg gereinigten und getrockneten Mikrogels fünf Mal in 20 ml THF (Tetrahydrofuran) eine Stunde suspendiert, zentrifugiert und der Überstand entfernt. Die Templatkonzentration im Überstand wurde mittels eines UV-Spectrometers (Spectramax Plus, Molecular Devices) gemessen. 50 mg extrahierten und getrockneten Mikrogels wurden in 5, 10 oder 20 ml einer Templatlösung in Methanol/Wasser (50:50, v/v) suspendiert bei einer BFA-Konzentration von 1 mM. Nach 20-stündiger Inkubation wurde die Suspension mittels Ultrazentrifugation (20.000 Upm, 35 Minuten) getrennt und die Menge an Templat im Überstand mittels UV-Spektroskopie quantifiziert.

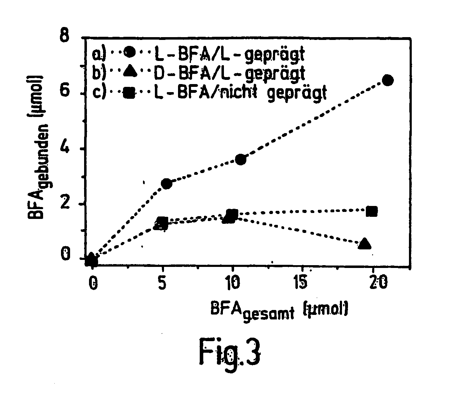

Der Nachweis der spezifischen Rückbindung des BFA-Templats wurde titrimetrisch mittels UV-Spektroskopie durchgeführt. Dazu wurde ein mit L-BFA geprägtes Mikrogel aus Poly (MAA-co-EGDMA) mit einem Monomer/Vernetzer-Anteil von 1:4 vorgelegt und (a) L-beziehungsweise (b) D-BFA zutitriert. Als Referenz diente das analoge nicht geprägte Mikrogel (c). Figur 3 fasst die Titrationsexperimente zusammen. In Figur 3 ist eine quantitativ deutlich stärkere Rückbindung für das Prägemolekül L-BFA im Vergleich zum D-Enantiomer zu sehen, was einer spezifischen Bindung entspricht. Die geringe Bindung von L-BFA im Fall des nicht geprägten Mikrogels (c) ist auf unspezifische Wechselwirkungen zurückzuführen.

Die Beispiele zeigen, dass aus Poly(MAA)-co-(EGDMA), Poly(EGDMA), Poly(MAA)-co-(DVB) und Poly(DVB) Mikrogele mittels Miniemulsionspolymerisation synthetisiert werden können und in vorzüglicher Weise ein direktes nicht-kovalentes molekulares Prägen einer chiralgeschützten Aminosäure erlauben. Die besten molekularen Wiedererkennungseigenschaften wies Poly(MAA)-co-(EGDMA) auf, insbesondere in einem molaren Verhältnis von 1:4 der Monomere. Die vorliegende einschrittige Verfahrensweise eröffnet daher neuartige Anwendungsmöglichkeiten molekularen Prägens. Die entwickelte Miniemulsionspolymerisation zur Herstellung molekular geprägter Polymere weist sowohl hinsichtlich der Ausbeute von Nanopartikeln im Verhältnis zu den Edukten als auch hinsichtlich der Rückgewinnung von Templaten hohe Effizienz auf, wobei die hergestellten Nanosphären beziehungsweise Nanopartikel eine große spezifische Oberfläche und kleine Gesamtgröße aufweisen. Die erfindungsgemäßen Nanopartikel können zum Erkennen und Binden bestimmter Substanzen in verdünnten Lösungen eingesetzt werden, zum Beispiel für das Abtrennen toxischer Agenzien. Katalytisch aktive Nanopartikel der Erfindung können in Suspension eingesetzt werden und stellen so die Möglichkeit zur Verfügung, leicht zugängliche katalytische Stellen auf kolloidaler Oberfläche bereitzustellen. Erfindungsgemäße Mikrogele können zum Beschichten von Sensoroberflächen oder Membranen eingesetzt werden. Die erfindungsgemäßen Mikrogele und Nanopartikel erweisen sich als besonders vorteilhaft insofern, als dass sie in geometrisch nicht limitierter Weise aufgebracht werden können, zum Beispiel auf analytische oder medizinische Vorrichtungen in beliebiger Geometrie.

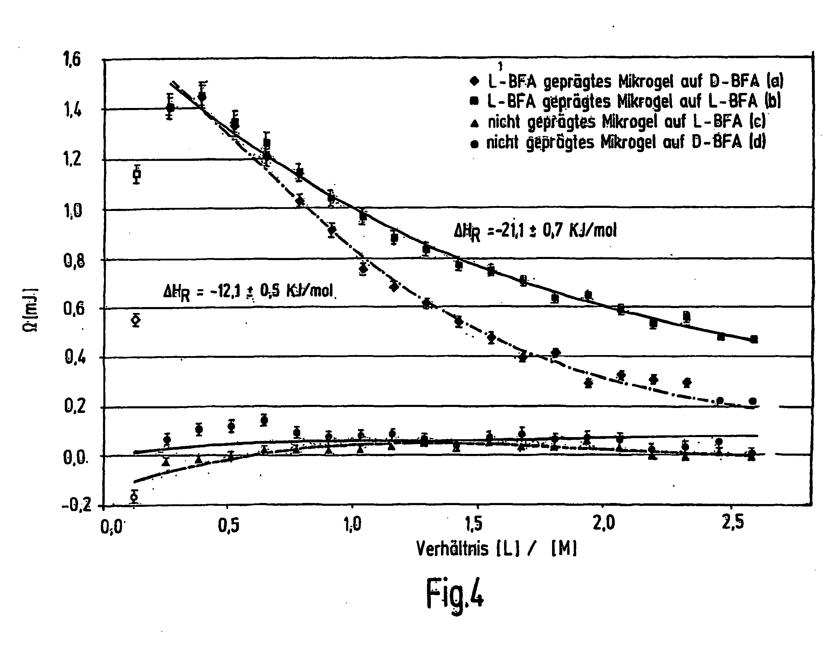

Einen weiteren Beweis für die spezifische Wiedererkennung leistet die isotherme Titrationsmikrokalorimetrie (ITC). Mit dieser kalorimetrischen Methode werden über definierte portionsweise Zugaben Reaktionspartner gemischt. Die dabei freiwerdenden Wärmen werden aufgezeichnet. Mit dieser Art der Durchführung der Experimente ist die direkte Bestimmung von Reaktionsenthalpien möglich. Die Reaktionsenthalpien geben Aufschluss über die Stärke einer Reaktion. Titriert man D- (Figur 4d) oder L-BFAhaltige Lösungen (Figur 4c) mit einem nicht geprägten Mikrogel erhält man Reaktionsenthalpien um ± 0 KJ/mol. Verwendet man im Gegensatz dazu ein L-BFA geprägtes Mikrogel und titiert damit L-beziehungsweise D-BFA, erhält man exotherme Reaktionsenthalpien. Bei dem Versuch mit L-BFA (Figur 4b) erhält man im Vergleich zu dem Versuch mit D-BFA (Figur 4a) eine um 57 % erhöhte Reaktionsenthalpie. Die Ergebnisse und die Versuchsparameter sind in der Figur 4 beziehungsweise in der Tabelle zusammengefasst.

### Tabelle

Dargestellt sind die Versuchsbedingungen (Reaktionspartner, Konzentrationsangaben) und die Ergebnisse (Reaktionsenthalpien) der mikrokalorimetrischen Untersuchungen an geprägten und nicht geprägten Mikrogelen.

| Reaktionspartner | Zugabe Mikrogel (mg) | Konz. BFA | Reaktionsenthalpie |
|---|---|---|---|
| | | µmol/l | ΔH_{R} (KJ/mol) |
| L-BFA geprägtes Mikrogel/L-BFA (b) | 20,5 | 770 | -21,1 ± 0,7 |
| L-BFA geprägtes Mikrogel/D-BFA (a) | 20,5 | 767 | -12,1 ± 0,5 |
| nicht geprägtes Mikrogel/L-BFA (c) | 20,5 | 770 | ≈ 0 |
| nicht geprägtes Mikrogel/D-BFA (d) | 20,5 | 767 | ≈ 0 |

Die Figur 4 zeigt die integrierte und normalisierten Isothermen der Titration von L-BFA geprägtem Mikrogel auf D-BFA (a) und L-BFA (b), sowie der Tittration von nicht geprägtem Mikrogel auf L-BFA (c) beziehungsweise D-BFA (d).

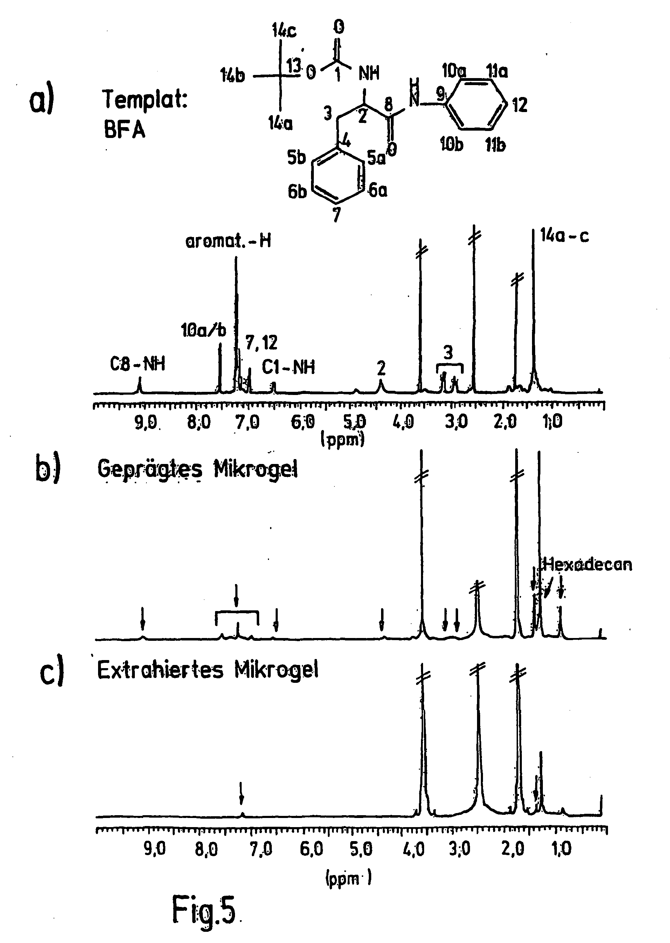

Figur 5 zeigt die ¹H-NMR-Spektren a) des reinen L-BFA, b) des L-BFA geprägten Mikrogels und c) des Mikrogels nach Extraktion des L-Templats. Sowohl die Bindung des Templats im geprägten Mikrogel als auch die Extraktion sind anhand der Intensität der NMR-Signale des L-BFA qualitativ eindeutig nachweisbar.

### Ausführungsbeispiel 8

### Herstellung von surfmerstabilisierten Latexpartikeln

50 mg des polymerisierbaren Emulgators p-(11-Acrylamido)-undecenoyloxyphenyl-dimethylsulfonium methylsulfat werden in 30 mL Wasser unter Rühren gelöst. Durch diese Lösung wird eine Stunde lang Argon geleitet. Daraufhin erfolgt unter Rühren die Zugabe von 1,8 mL Methylmethacrylat (MMA). Die entstandene Emulsion wird auf 60°C erwärmt. Durch Zugabe von 10 mg 2,2'-Azobis (2-Amidinopropan) dihydrochlorid wird die Copolymerisation von dem auch als Emulgator wirkenden p-(11-Acrylamido)-undecenoyloxyphenyl-dimethylsulfoniummethylsulfat und MMA gestartet. Nach 5 h wurde die Reaktion beendet, die Latexlösung abgekühlt und die Teilchengröße mittels Dynamischer Lichtstreuung bestimmt. Der Mittelwert der Größenverteilung (bezogen auf die Intensitätsverteilung) befindet sich zwischen 140 und 150 nm.

Das so erhaltene Polymer wird anschließend mit molekülspezifischen Erkennungsstellen versehen, zum Beispiel Streptavidin-Gruppen.

Zur Abtrennung von überschüssigen Monomeren und I-nitiatorresten wurde die Latexlösung dreimal zentrifugiert, der Überstand verworfen und die abzentrifugierten Polymerpartikel in Wasser redispergiert.

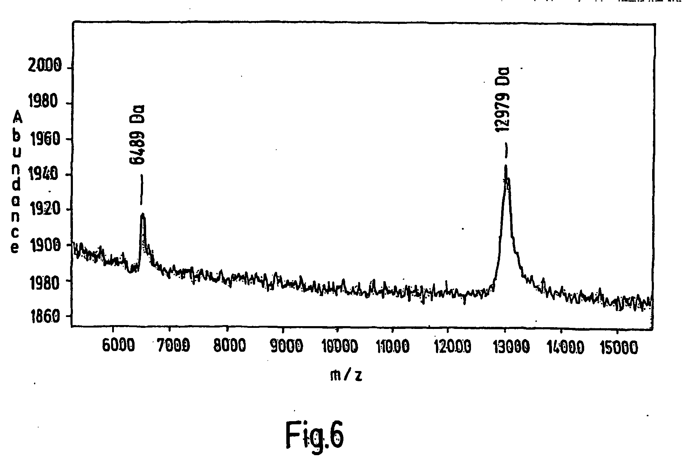

20 µL der gereinigten Latexlösung wurden in 1 mL Phosphatpuffer (pH 7,8) aufgenommen und mit 10 µL Streptavidinlösung (cₛₐᵥ = 13,4 nmol/mL) versetzt. Die so hergestellte Reaktionslösung wurde 2 h bei Raumtemperatur geschüttelt und anschließend zentrifugiert. Zur Abtrennung von nicht immobilisiertem Streptavidin wurde die Latexlösung dreimal zentrifugiert, der Überstand verworfen und die abzentrifugierten Polymerpartikel in Wasser redispergiert. 1 µL der hergestellten Suspension wurde zusammen mit Sinapinsäurelösung auf einen MALDI-TOF-MS-Probenträger (Matrix Assisted Laserdesorption Ionisation-Time of Flight-Mass Spectrometry) gebracht und analysiert. Im Massenspektrum der Probe (Figur 6) ist bei einem m/z Verhältnis von 12979 Da/Elementarladung ein Signal vorhanden, welches dem Streptavidin [M+1]⁺-Signal zugeordnet werden kann (6489 Da = [M+1]²⁺).

Auf den Partikeln ist demnach Streptavidin durch das polymerisierbare Aktivester-Tensid gebunden.

## Patentansprüche

1. Nanopartikel mit einem Durchmesser von 20 bis 500 nm, die molekülspezifische Erkennungsstellen aufweisen, die eine spezifische Wechselwirkung zwischen den Nanopartikeln und Zielmolekülen erlauben, wobei die Nanopartikel erhältlich sind durch Herstellen einer Miniemulsion, umfassend zwei nicht miteinander mischbare Phasen, wobei die Miniemulsion
a) mindestens ein Monomer,
b) mindestens ein Templat oder mindestens ein oberflächenaktives polymerisierbares Monomer als ein Molekülspezifität verleihendes Agens, wobei das oberflächenaktive polymerisierbare Monomer ein Vinyl-Monomer mit hydrophoben Segmenten und hydrophilen reaktiven Endgruppen ist und wobei die hydrophilen, reaktiven Endgruppen Sulfonium-aktivierte Ester- oder Succinimidester-Gruppen sind,
c) ein stabilisierendes Mittel, und
d) mindestens einen Emulgator enthält
und Durchführen einer Miniemulsionspolymerisation und wobei die molekülspezifische Erkennungsstellen erhältlich sind durch Einpolymerisieren von Templaten und anschließendes Auswaschen oder durch Einpolymerisieren von oberflächenaktiven polymerisierbaren Monomeren.

2. Nanopartikel nach Anspruch 1, wobei die Miniemulsion mindestens einen Initiator enthält.

3. Nanopartikel nach einem der Ansprüche 1 oder 2, wobei die Miniemulsion mindestens einen Vernetzer enthält.

4. Nanopartikel nach einem der Ansprüche 1 bis 3, erhältlich durch
a) Herstellen eines Monomer-haltigen Gemisches aus mindestens einem Monomer sowie mindestens einem Molekülspezifität verleihenden Agens,
b) Herstellen einer Emulgator-haltigen Lösung,
c) Versetzen des Monomer-haltigen Gemisches und/oder der Emulgator-haltigen Lösung mit einem stabilisierenden Mittel und
d) Durchführen einer Miniemulsionspolymerisation durch Vereinigen der Emulgator-haltigen Lösung mit dem Monomer-haltigen Gemisch.

5. Nanopartikel nach Anspruch 4, wobei in Schritt a) das Monomer-haltige Gemisch zusätzlich aus mindestens einem Initiator hergestellt wird.

6. Nanopartikel nach einem der Ansprüche 4 oder 5, wobei in Schritt a) das Monomer-haltige Gemisch zusätzlich aus mindestens einem Vernetzer hergestellt wird.

7. Nanopartikel nach einem der Ansprüche 1 bis 6, wobei die Miniemulsionspolymerisation durch thermisches oder photochemisches Starten eingeleitet wird.

8. Nanopartikel nach einem der vorhergehenden Ansprüche, wobei das mindestens eine Monomer mindestens eine funktionelle Gruppe aufweist, die zur Interaktion mit einem Templat befähigt.

9. Nanopartikel nach einem der vorhergehenden Ansprüche, wobei das Templat nach dem Miniemulsionspolymerisationsvorgang aus den gebildeten Nanopartikeln herausgelöst wird.

10. Nanopartikel nach einem der vorherigen Ansprüche, wobei das Templat eine Aminosäure, Aminosäurederivat, Peptid, Protein, Antikörper, Glycoprotein, Cofaktor, Vitamin, Biotin, Enzym, Nucleinsäure, Bakterium, Virus, Viroid, Prion, Zellorganell- oder Bestandteil, Proteoglycan, Lipid, Lektin, Kohlenhydrat, Monosaccharid, Oligosaccharid, Polysaccharid, Glycosaccharid oder heterozyklische Verbindung ist.

11. Nanopartikel nach einem der Ansprüche 1 bis 7, wobei das oberflächenaktive polymerisierbare Monomer amphiphil ist.

12. Nanopartikel nach Anspruch 11, wobei das oberflächenaktive polymerisierbare Monomer molekülspezifische Erkennungsstellen trägt.

13. Nanopartikel nach Anspruch 11 oder 12, wobei das oberflächenaktive polymerisierbare Monomer mittels mindestens einer chemischen Reaktion mit molekülspezifischen Erkennungsstellen versehen wird.

14. Nanopartikel nach einen der Ansprüche 11 bis 13, wobei das Vinyl-Monomer ein Methacryl-, Acryl-, Acrylamid-, Styrol- oder Styrolsulfon-Monomer ist.

15. Nanopartikel nach Anspruch 14, wobei die hydrophoben Segmente Methylen, Ethylenoxid oder Butylenoxid umfassen.

16. Nanopartikel nach einem der Ansprüche 14 bis 15, wobei das oberflächenaktive polymerisierbare Monomer ein Vinylalkyloyloxyphenyldimethylsulfoniumsalz ist.

17. Nanopartikel nach einem der vorhergehenden Ansprüche, wobei das mindestens eine Monomer ein hydrophiles Monomer ist.

18. Nanopartikel nach einem der vorhergehenden Ansprüche, wobei das mindestens eine Monomer ein hydrophobes Monomer ist.

19. Nanopartikel nach einem der vorhergehenden Ansprüche, wobei das die Molekülspezifität verleihende Agens wasserlöslich oder wasserunlöslich ist.

20. Nanopartikel nach einem der Ansprüche 1 bis 19, wobei das stabilisierende Mittel ein hydrophiles oder hydrophobes Agens ist.

21. Nanopartikel nach Anspruch 20, wobei das hydrophobe Agens Hexadecan ist.

22. Nanopartikel nach einem der vorhergehenden Ansprüche, wobei der Vernetzer ein hydrophober oder hydrophiler Vernetzer ist.

23. Nanopartikel nach Anspruch 22, wobei der Vernetzer ein Acrylatderivat oder ein Styrolderivat ist.

24. Nanopartikel nach Anspruch 23, wobei das Acrylatderivat Ethylenglycoldimethacrylat (EGDMA), N,N'-Methylenbisacrylamid, Trimethylolpropantrimethacrylat oder Bisphenol A ist.

25. Nanopartikel nach Anspruch 23, wobei das Styrolderivat Divinylbenzol oder Diallylweinsäurediamid ist.

26. Nanopartikel nach einem der vorhergehenden Ansprüche, wobei der Initiator ein Azoderivat oder ein Peroxid ist.

27. Nanopartikel nach Anspruch 26, wobei das Azoderivat α,α'-Azoisobutyronitril, 2,2'-Azobis(2-methylbutyronitril) oder 1,1'-Azodi(hexahydrobenzonitril) ist.

28. Nanopartikel nach Anspruch 26, wobei das Peroxid, zum Beispiel Kaliumperoxodisulfat oder Bis(α-α-dimethylbenzyl)peroxid ist.

29. Nanopartikel nach einem der vorhergehenden Ansprüche, wobei der Emulgator ein ionischer Emulgator, ein kationischer Emulgator oder ein nichtionischer Emulgator ist.

30. Nanopartikel nach Anspruch 29, wobei der ionische Emulgator Natriumdodecylsulfat oder Hexadecylsulfonsäure ist.

31. Nanopartikel nach Anspruch 29, wobei der kationische Emulgator Hexadecyltrimethylammoniumbromid oder Dodecylpyridiniumchlorid ist.

32. Nanopartikel nach einem der vorhergehenden Ansprüche, wobei die Nanopartikel in Form von kolloidalen Polymeren (Latices) oder geträgert vorliegen.

33. Nanopartikel nach Anspruch 32, wobei die Latices vernetzte Latices (Mikrogele) sind.

34. Verfahren zur Herstellung von Nanopartikeln nach einem der vorhergehenden Ansprüche, die molekülspezifische Erkennungsstellen, die eine spezifische Wechselwirkung zwischen dem Nanopartikel und Zielmolekülen erlauben, aufweisen, wobei eine zwei nicht miteinander mischbare Phasen umfassende Miniemulsion hergestellt wird, enthaltend
a) mindestens ein Monomer,
b) mindestens ein Templat oder mindestens ein oberflächenaktives polymerisierbares Monomer als ein Molekülspezifität verleihendes Agens, wobei das oberflächenaktive polymerisierbare Monomer ein Vinyl-Monomer mit hydrophoben Segmenten und hydrophilen reaktiven Endgruppen ist und wobei die hydrophilen, reaktiven Endgruppen Sulfonium-aktivierte Ester- oder Succinimid-ester-Gruppen sind,
c) ein stabilisierendes Mittel, und
d) mindestens einen Emulgator
und anschließend eine Miniemulsionspolymerisation durchgeführt wird.

35. Verfahren nach Anspruch 34, wobei die Miniemulsion mindestens einen Initiator enthält.

36. Verfahren nach einem der Ansprüche 34 oder 35, wobei die Miniemulsion mindestens einen Vernetzer enthält.

37. Verfahren zur Herstellung molekülspezifische Erkennungsstellen aufweisender Nanopartikel nach einem der Ansprüche 34 bis 36, wobei ein Monomer-haltiges Gemisch aus mindestens einem Monomer, mindestens einem Molekülspezifität verleihenden Agens sowie eine Emulgator-haltige Lösung hergestellt werden, wobei das Monomer-haltige Gemisch und/oder die Emulgator-haltige Lösung mit einem stabilisierenden Mittel versetzt und wobei eine Miniemulsionspolymerisation durch Vereinigen der Emulgator-haltigen Lösung mit dem Monomer-haltigen Gemisch durchgeführt wird.

38. Verfahren nach Anspruch 37, wobei das Monomer-haltige Gemisch zusätzlich aus mindestens einem Initiator hergestellt wird.

39. Verfahren nach einem der Ansprüche 37 oder 38, wobei das Monomer-haltige Gemisch zusätzlich aus mindestens einem Vernetzer hergestellt wird.

40. Verfahren nach einem der Ansprüche 34 bis 39, wobei die Miniemulsionspolymerisation eine hydrophobe-Phase-in-hydrophile-Phase-Miniemulsionspolymerisation ist.

41. Verfahren nach Anspruch 40, wobei die hydrophobe-Phase-in-hydrophile-Phase-Miniemulsionspolymerisation eine Öl-in-Wasser-Miniemulsionspolymensation ist.

42. Verfahren nach einem der Ansprüche 34 bis 39, wobei die Miniemulsionspolymerisation eine hydrophile-Phase-in-hydrophobe-Phase-Miniemulsionspolymerisation ist.

43. Verfahren nach Anspruch 42, wobei die hydrophile-Phase-inhydrophobe-Phase-Miniemulsionspolymerisation eine Wasser-in-Öl-Miniemulsionspolymerisation ist.

44. Verfahren nach einem der Ansprüche 34 bis 43, wobei das Molekülspezifität verleihende Agens ein Templat ist und dieses nach der Miniemulsionspolymerisation aus den gebildeten Nanopartikeln herausgelöst wird.

45. Verfahren nach einem der Ansprüche 34 bis 44, wobei das Monomer ein amphiphiles Monomer ist.

46. Verfahren nach einem der Ansprüche 34 bis 45, wobei die Miniemulsionspolymerisation thermisch oder photochemisch gestartet wird.

47. Verfahren nach einem der Ansprüche 40 oder 41, wobei das stabilisierende Mittel ein hydrophobes Agens ist.

48. Verfahren nach Anspruch 47, wobei das hydrophobe Agens Hexadecan ist.

49. Verfahren nach einem der Ansprüche 42 oder 43, wobei das stabilisierende Mittel ein hydrophiles Agens ist.

50. Partikulär aufgebauter Film oder partikulär aufgebaute Membran, hergestellt durch Abscheiden von Nanopartikeln mit molekülspezifischen Erkennungsstellen, die eine spezifische Wechselwirkung zwischen Nanopartikeln und Zielmolekülen erlauben, nach einem der Ansprüche 1 bis 32 aus Latices.

51. Film oder Membran nach Anspruch 50, wobei die Latices Mikrogele gemäß Anspruch 33 sind.

52. Film oder Membran nach Anspruch 50 oder 51, wobei dieser eine Dicke von 10 nm bis 1000 µm aufweist.

53. Film oder Membran nach Anspruch 52, wobei dieser eine Dicke von 10 nm bis 500 µm aufweist.

54. Film oder Membran nach einem der Ansprüche 50 bis 53, der auf einem Substrat geträgert ist.

55. Film oder Membran nach einem der Ansprüche 50 bis 53, wobei das Substrat eine Sensoroberfläche, ein Stent, eine Filtermembran, ein Hohlfasermodul, ein Metall, ein Halbmetall, ein Metalloxid, Glas, Papier, ein Kunststoff, Nylon, Polyphenyloxid, verflochtene oder verfilzte Fasern, mikroporöse Keramiken, Graphit, rostfreier Stahl, eine Membran, ein Filter, eine Folie oder ähnliches ist.

56. Film oder Membran nach Anspruch 55, wobei das Halbmetall Silizium ist.

57. Film oder Membran nach Anspruch 55, wobei der Kunststoff Polyester, Polyarylsulfon, Polyethersulfon, Polyphenylsulfon, Polyvinylsulfon, Polystyrol, Polycarbonat, Polyethylen, Polypropylen, Polyacetylen, Polyvinylpiridin, Polyvinylidenfluorid, Polyvinylchlorid, Polyvinylpyrolidon, Polytetrafluorethylen oder ein Derivat davon ist.

58. Film oder Membran nach einem der Ansprüche 50 bis 57, wobei der Film oder die Membran mechanisch stabil und/oder permeabel und/oder regenerierbar sind.

59. Verfahren zur Herstellung eines Films oder einer Membran nach einem der Ansprüche 50 bis 58, wobei mindestens ein Latex nach Anspruch 32 oder 33 mindestens einer Filtration sowie einer Trocknung ausgesetzt und ein partikulärer ein-oder mehrschichtiger Film oder Membran erhalten wird.

## Claims

1. Nanoparticles with a diameter of 20 to 500 nm, which have molecule-specific recognition sites which allow a specific interaction between the nanoparticles and target molecules, the nanoparticles being obtainable by production of a miniemulsion, comprising two phases which are not miscible with each other, the miniemulsion containing
a) at least one monomer,
b) at least one template or at least one surface-active polymerisable monomer as an agent which imparts molecule specificity, the surface-active polymerisable monomer being a vinyl monomer with hydrophobic segments and hydrophilic reactive end groups and the hydrophilic, reactive end groups being sulphonium-activated ester or succinimide ester groups,
c) a stabilising agent, and
d) at least one emulsifier
and implementation of a miniemulsion polymerisation, and the molecule-specific recognition sites being obtainable by polymerisation therein of templates and subsequent washing-out or by polymerisation therein of surface-active polymerisable monomers.

2. Nanoparticles according to claim 1, the miniemulsion containing at least one initiator.

3. Nanoparticles according to one of the claims 1 or 2, the miniemulsion containing at least one crosslinker.

4. Nanoparticles according to one of the claims 1 to 3, obtainable by
a) production of a monomer-containing mixture comprising at least one monomer and also at least one agent which imparts molecule specificity,
b) production of an emulsifier-containing solution,
c) mixing of the monomer-containing mixture and/or the emulsifier-containing solution with a stabilising medium and
d) implementation of a miniemulsion polymerisation by combining the emulsifier-containing solution with the monomer-containing mixture.

5. Nanoparticles according to claim 4, the monomer-containing mixture being produced, in step a), in addition from at least one initiator.

6. Nanoparticles according to one of the claims 4 or 5, the monomer-containing mixture being produced, in step a), in addition from at least one crosslinker.

7. Nanoparticles according to one of the claims 1 to 6, the miniemulsion polymerisation being initiated by thermal or photochemical starting.

8. Nanoparticles according to one of the preceding claims, the at least one monomer having at least one functional group which is capable of interaction with a template.

9. Nanoparticles according to one of the preceding claims, the template being extracted after the miniemulsion polymerisation process from the formed nanoparticles.

10. Nanoparticles according to one of the preceding claims, the template being an amino acid, amino acid derivative, peptide, protein, antibody, glycoprotein, cofactor, vitamin, biotin, enzyme, nucleic acid, bacterium, virus, viroid, prion, cell organelle or component, proteoglycan, lipid, lectin, carbohydrate, monosaccharide, oligosaccharide, polysaccharide, glycosaccharide or heterocyclic compound.

11. Nanoparticles according to one of the claims 1 to 7, the surface-active polymerisable monomer being amphiphilic.

12. Nanoparticles according to claim 11, the surface-active polymerisable monomer carrying molecule-specific recognition sites.

13. Nanoparticles according to claim 11 or 12, the surface-active polymerisable monomer being provided by means of at least one chemical reaction with molecule-specific recognition sites.

14. Nanoparticles according to one of the claims 11 to 13, the vinyl monomer being a methacryl-, acryl-, acrylamide-, styrene- or styrenesulphone monomer.

15. Nanoparticles according to claim 14, the hydrophobic segments comprising methylene, ethylene oxide or butylene oxide.

16. Nanoparticles according to one of the claims 14 to 15, the surface-active polymerisable monomer being a vinylalkyloyloxyphenyldimethylsulphonium salt.

17. Nanoparticles according to one of the preceding claims, the at least one monomer being a hydrophilic monomer.

18. Nanoparticles according to one of the preceding claims, the at least one monomer being a hydrophobic monomer.

19. Nanoparticles according to one of the preceding claims, the agent which imparts the molecule specificity being water-soluble or water-insoluble.

20. Nanoparticles according to one of the claims 1 to 19, the stabilising agent being a hydrophilic or hydrophobic agent.

21. Nanoparticles according to claim 20, the hydrophobic agent being hexadecane.

22. Nanoparticles according to one of the preceding claims, the crosslinker being a hydrophobic or hydrophilic crosslinker.

23. Nanoparticles according to claim 22, the crosslinker being an acrylate derivative or a styrene derivative.

24. Nanoparticles according to claim 23, the acrylate derivative being ethyleneglycoldimethacrylate (EGDMA), N,N'-methylenebisacrylamide, trimethylolpropanetrimethacrylate or bisphenol A.

25. Nanoparticles according to claim 23, the styrene derivative being divinylbenzene or diallyl tartaric acid diamide.

26. Nanoparticles according to one of the preceding claims, the initiator being an azo derivative or a peroxide.

27. Nanoparticles according to claim 26, the azo derivative being α-α'-azoisobutyronitrile, 2,2'-azobis(2-methylbutyronitrile) or 1,1'-azodi(hexahydrobenzonitrile).

28. Nanoparticles according to claim 26, the peroxide being for example potassium peroxodisulphate or bis(α-α-dimethylbenzyl) peroxide.

29. Nanoparticles according to one of the preceding claims, the emulsifier being an ionic emulsifier, a cationic emulsifier or a nonionic emulsifier.

30. Nanoparticles according to claim 29, the ionic emulsifier being sodium dodecylsulphate or hexadecylsulphonic acid.

31. Nanoparticles according to claim 29, the cationic emulsifier being hexadecyltrimethyl ammonium bromide or dodecylpyridinium chloride.

32. Nanoparticles according to one of the preceding claims, the nanoparticles being present in the form of colloidal polymers (latices) or carried.

33. Nanoparticles according to claim 32, the latices being crosslinked latices (microgels).

34. Method for the production of nanoparticles according to one of the preceding claims, which have molecule-specific recognition sites which allow a specific interaction between the nanoparticle and target molecules, a miniemulsion which comprises two phases which are not miscible with each other being produced, containing
a) at least one monomer,
b) at least one template or at least one surface-active polymerisable monomer as an agent which imparts molecule specificity, the surface-active polymerisable monomer being a vinyl monomer with hydrophobic segments and hydrophilic reactive end groups and the hydrophilic, reactive end groups being sulphonium activated ester or succinimide ester groups,
c) a stabilising medium, and
d) at least one emulsifier
and subsequently a miniemulsion polymerisation being implemented.

35. Method according to claim 34, the miniemulsion containing at least one initiator.

36. Method according to one of the claims 34 or 35, the miniemulsion containing at least one crosslinker.

37. Method for the production of nanoparticles which have molecule specific recognition sites according to one of the claims 34 to 36, a monomer-containing mixture comprising at least one monomer, at least one agent which imparts molecule specificity and also an emulsifier-containing solution being produced, the monomer-containing mixture and/or the emulsifier-containing solution being mixed with a stabilising medium and a miniemulsion polymerisation being implemented by combining the emulsifier-containing solution with the monomer-containing mixture.

38. Method according to claim 37, the monomer-containing mixture being produced in addition from at least one initiator.

39. Method according to one of the claims 37 or 38, the monomer-containing mixture being produced in addition from at least one crosslinker.

40. Method according to one of the claims 34 to 39, the miniemulsion polymerisation being a hydrophobic phase-in-hydrophilic phase miniemulsion polymerisation.

41. Method according to claim 40, the hydrophobic phase-in-hydrophilic phase miniemulsion polymerisation being an oil-in-water miniemulsion polymerisation.

42. Method according to one of the claims 34 to 39, the miniemulsion polymerisation being a hydrophilic phase-in-hydrophobic phase miniemulsion polymerisation.

43. Method according to claim 42, the hydrophilic phase-in-hydrophobic phase miniemulsion polymerisation being a water-in-oil miniemulsion polymerisation.

44. Method according to one of the claims 34 to 43, the agent which imparts molecule specificity being a template and this being extracted after the miniemulsion polymerisation from the formed nanoparticles.

45. Method according to one of the claims 34 to 44, the monomer being an amphiphilic monomer.

46. Method according to one of the claims 34 to 45, the miniemulsion polymerisation being started thermally or photochemically.

47. Method according to one of the claims 40 or 41, the stabilising medium being a hydrophobic agent.

48. Method according to claim 47, the hydrophobic agent being hexadecane.

49. Method according to one of the claims 42 or 43, the stabilising medium being a hydrophilic agent.

50. Particulate-constructed film or particulate-constructed membrane, produced by deposition of nanoparticles with molecule-specific recognition sites which allow a specific interaction between nanoparticles and target molecules, according to one of the claim 1 to 32 from latices.

51. Film or membrane according to claim 50, the latices being microgels according to claim 33.

52. Film or membrane according to claim 50 or 51, this having a thickness of 10 nm to 1000 µm.

53. Film or membrane according to claim 52, this having a thickness of 10 nm to 500 µm.

54. Film or membrane according to one of the claims 50 to 53, which is carried on a substrate.

55. Film or membrane according to one of the claims 50 to 53, the substrate being a sensor surface, a stent, a filter membrane, a hollow fibre module, a metal, a semimetal, a metal oxide, glass, paper, a plastic material, nylon, polyphenyloxide, braided or felted fibres, microporous ceramics, graphite, stainless steel, a membrane, a filter, a foil or the like.

56. Film or membrane according to claim 55, the semimetal being silicon.

57. Film or membrane according to claim 55, the plastic material being polyester, polyarylsulphone, polyethersulphone, polyphenylsulphone, polyvinylsulphone, polystyrene, polycarbonate, polyethylene, polypropylene, polyacetylene, polyvinylpyridine, polyvinylidine fluoride, polyvinylchloride, polyvinylpyrolidone, polytetrafluoroethylene or a derivative thereof.

58. Film or membrane according to one of the claims 50 to 57, the film or the membrane being mechanically stable and/or permeable and/or regeneratable.

59. Method for the production of a film or of a membrane according to one of the claims 50 to 58, at least one latex according to claim 32 or 33 being subjected at least to a filtration and also to a drying and a particulate single- or multilayer film or membrane being obtained.

## Revendications

1. Nanoparticules ayant un diamètre de 20 nm à 500 nm, qui présentent des points de reconnaissance spécifiques de molécules permettant une interaction spécifique entre les nanoparticules et des molécules cibles, les nanoparticules pouvant être obtenues par préparation d'une mini-émulsion renfermant deux phases non miscibles entre elles, la mini-émulsion renfermant :
a) au moins un monomère,
b) au moins un calibre de formulation ou au moins un monomère polymérisable tensioactif en tant qu'agent conférant une spécificité moléculaire, le monomère polymérisable tensioactif étant un monomère vinylique comportant des segments hydrophobes et des groupes terminaux réactifs hydrophiles, et les groupes terminaux réactifs hydrophiles étant des groupes ester activés par un radical sulfonium ou des groupes succiminides ester,
c) un agent stabilisant, et
d) au moins un agent émulsifiant,
et mise en oeuvre d'une polymérisation de la mini-émulsion, les points de reconnaissance spécifiques de molécules pouvant être obtenus par polymérisation de calibres de formulation puis lavage, ou par polymérisation de monomères polymérisables tensioactifs.

2. Nanoparticules selon la revendication 1,
dans lesquelles
la mini-émulsion renferme au moins un agent initiateur.

3. Nanoparticules selon la revendication 1 ou 2,
dans lesquelles
la mini-émulsion renferme au moins un agent de réticulation.

4. Nanoparticules selon l'une des revendications 1 à 3, pouvant être obtenues par :
a) préparation d'un mélange monomère d'au moins un monomère et d'au moins un agent de nature à conférer une spécificité de moléculaire,
b) préparation d'une solution renfermant un agent émulsifiant,
c) combinaison du mélange monomère et/ou de la solution renfermant un agent émulsifiant avec un agent stabilisant et,
d) mise en oeuvre d'une polymérisation de la mini-émulsion par combinaison de la solution renfermant l'agent émulsifiant avec le mélange monomère,

5. Nanoparticules selon la revendication 4,
dans lesquelles
dans l'étape a), on prépare le mélange monomère en mettant également en oeuvre au moins un agent initiateur.

6. Nanoparticules selon l'une des revendications 4 et 5,
dans lesquelles,
dans l'étape a), on prépare le mélange monomère en mettant également en oeuvre au moins un agent de réticulation.

7. Nanoparticules selon l'une des revendications 1 à 6,
dans lesquelles
la polymérisation de la mini-émulsion est déclenchée par démarrage thermique ou photochimique.

8. Nanoparticules selon l'une des revendications précédentes,
dans lesquelles
le ou les monomère(s) contient/contiennent au moins un groupe fonctionnel apte à interagir avec un calibre de formulation.

9. Nanoparticules selon l'une des revendications précédentes,
dans lesquelles
le calibre de formulation est éliminé des nanoparticules formées par dissolution après le processus de polymérisation de la mini-émulsion.

10. Nanoparticules selon l'une des revendications précédentes,
dans lesquelles
le calibre de formulation est un acide aminé, un dérivé d'acide aminé, un peptide, une protéine, un anticorps, une glycoprotéine, un cofacteur, une vitamine, une biotine, une enzyme, un acide nucléique, une bactérie, un virus, un viroïde, un prion, un élément ou constituant une organite cellulaire, un protéoglycane, un lipide, une lectine, un hydrate de carbone, un monosaccharide, un oligosaccharide, un polysaccharide, un glycosaccharide ou un composé hétérocyclique.

11. Nanoparticules selon l'une des revendications 1 à 7,
dans lesquelles
le monomère polymérisable tensioactif est amphiphile.

12. Nanoparticules selon la revendication 11,
dans lesquelles
le monomère polymérisable tensioactif porte des points de reconnaissance spécifiques de molécules.

13. Nanoparticules selon la revendication 11 ou 12,
dans lesquelles
le monomère polymérisable tensioactif est pourvu de points de reconnaissance spécifiques de molécules par au moins une réaction chimique.

14. Nanoparticules selon l'une des revendications 11 à 13,
dans lesquelles
le monomère vinylique est un monomère méthacrylique, acrylique, d'acrylamide, styrénique ou styrène sulfoniques.

15. Nanoparticules selon la revendication 14,
dans lesquelles
les segments hydrophobes renferment du méthylène de l'oxyde d'éthylène ou de l'oxyde de butylène.

16. Nanoparticules selon l'une des revendications 14 et 15,
dans lesquelles
le monomère polymérisable tensioactif est un sel de vinylalkyloyloxyphényldiméthylsufonium.

17. Nanoparticules selon l'une des revendications précédentes,
dans lesquelles
le ou les monomères est/sont un ou des monomère(s) hydrophile(s).

18. Nanoparticules selon l'une des revendications précédentes,
dans lesquelles
le ou les monomères est/sont un ou des monomère(s) hydrophobe(s).

19. Nanoparticules selon l'une des revendications précédentes,
dans lesquelles
l'agent conférant la spécificité moléculaire est soluble ou insoluble dans l'eau.

20. Nanoparticules selon l'une des revendications 1 à 19,
dans lesquelles
l'agent de stabilisation est un agent hydrophile ou hydrophobe.

21. Nanoparticules selon la revendication 20,
dans lesquelles
l'agent hydrophobe est l'hexadécane.

22. Nanoparticules selon l'une des revendications précédentes,
dans lesquelles
l'agent de réticulation est un agent de réticulation hydrophobe ou hydrophile.

23. Nanoparticules selon la revendication 22,
dans lesquelles
l'agent de réticulation et un dérivé d'acrylate ou de styrène.

24. Nanoparticules selon la revendication 23,
dans lesquelles
le dérivé d'acrylate est le diméthacrylate d'éthylèneglycol (EGDMA), le N,N'-méthylènebisacrylamide, le triméthacrylate de triméthylolpropane ou le bisphénol A.

25. Nanoparticules selon la revendication 23,
dans lesquelles
le dérivé de styrène est le divinylbenzène ou le diamide d'acide diallyltar.

26. Nanoparticules selon l'une des revendications précédentes,
dans lesquelles
l'agent initiateur est un dérivé azoïque ou un peroxyde.

27. Nanoparticules selon la revendication 26,
dans lesquelles
le dérivé d'azoïque est 1'α,α'-azoisobutyronitrile, le 2,2'-azobis(2-méthylbutyronitrile) ou le 1,1'-azodi(hexahydrobenzonitrile).

28. Nanoparticules selon la revendication 26,
dans lesquelles
le peroxyde est par exemple du peroxodisulfate de potassium ou du bis(a-a-diméthylbenzyl) peroxyde.

29. Nanoparticules selon l'une des revendications précédentes,
dans lesquelles
l'agent émulsifiant est un agent émulsifiant ionique un agent émulsifiant cationique ou un agent émulsifiant non ionique.

30. Nanoparticules selon la revendication 29,
dans lesquelles
l'agent émulsifiant ionique est du dodécylsulfate de sodium ou de l'acide hexadécylsulfonique.

31. Nanoparticules selon la revendication 29,
dans lesquelles
l'agent émulsifiant cationique est du bromure d'hexadécyltriméthylammonium ou du chlorure de dodécylpyridinium.

32. Nanoparticules selon l'une des revendications précédentes,
dans lesquelles
les nanoparticules se présentent sous la forme de polymères colloïdaux (latex) ou sont portées par un support.

33. Nanoparticules selon la revendication 32,
dans lesquelles
les latex sont des latex réticulés (microgels).

34. Procédé de préparation de nanoparticules selon l'une des revendications précédentes, qui présentent des points de reconnaissance spécifiques de molécules qui permettent une interaction spécifique entre les nanoparticules et des molécules cibles, selon lequel on prépare une mini-émulsion renfermant deux phases non miscibles l'une avec l'autre, renfermant :
a) au moins un monomère,
b) au moins un calibre de formulation ou au moins un monomère polymérisable tensioactif en tant qu'agent conférant une spécificité moléculaire, le monomère polymérisable tensioactif étant un monomère vinylique comportant des segments hydrophobes et des groupes terminaux réactifs hydrophiles, et les groupes terminaux réactifs hydrophiles étant des groupes ester activés par un radical sulfonium ou des groupes succinimide ester,
c) un agent de stabilisation, et
d) au moins un agent émulsifiant, et on met ensuite en oeuvre une polymérisation de la mini-émulsion.

35. Procédé selon la revendication 34,
selon lequel
la mini-émulsion renferme au moins un agent initiateur.

36. Procédé selon l'une des revendications 34 et 35,
selon lequel
la mini-émulsion renferme au moins un agent de réticulation.

37. Procédé de préparation de nanoparticules présentant des points de reconnaissance spécifiques de molécules selon l'une des revendications 34 à 35,
selon lequel
on prépare un mélange monomère d'au moins un monomère, d'au moins un agent conférant une spécificité moléculaire et d'une solution renfermant un agent émulsifiant, le mélange monomère et/ou la solution renfermant l'agent émulsifiant étant combiné avec un agent de stabilisation, et une polymérisation de la mini-émulsion étant mise en oeuvre par combinaison de la solution renfermant l'agent émulsifiant avec le mélange monomère.

38. Procédé selon la revendication 37,
selon lequel
le mélange monomère est en outre préparé en mettant en oeuvre au moins un agent initiateur.

39. Procédé selon l'une des revendications 37 et 38,
selon lequel
le mélange monomère est en outre préparé en mettant en oeuvre un agent de réticulation.

40. Procédé selon l'une des revendications 34 à 39,
selon lequel
la polymérisation de la mini-émulsion est une polymérisation en phase hydrophobe dans une phase hydrophile.

41. Procédé selon la revendication 40,
selon lequel
la polymérisation de la mini-émulsion en phase hydrophobe dans une phase hydrophile est une polymérisation huile dans l'eau.

42. Procédé selon l'une des revendications 34 à 39,
selon lequel
la polymérisation de la mini-émulsion est une polymérisation en phase hydrophile dans une phase hydrophobe.

43. Procédé selon la revendication 42,
selon lequel
la polymérisation de la mini-émulsion en phase hydrophile dans une phase hydrophobe est une polymérisation eau dans l'huile.

44. Procédé selon l'une des revendications 34 à 43,
selon lequel
l'agent conférant une spécificité moléculaire est un calibre de formulation qui est éliminé des nanoparticules formées par dissolution après la polymérisation de la mini-émulsion.

45. Procédé selon l'une des revendications 34 à 44,
selon lequel
le monomère est un monomère amphiphile.

46. Procédé selon l'une des revendications 34 à 45,
selon lequel
la polymérisation de la mini-émulsion est déclenchée par voie thermique ou photochimique.

47. Procédé selon l'une des revendications 40 et 41,
selon lequel
l'agent de stabilisation est un agent hydrophobe.

48. Procédé selon la revendication 47,
selon lequel
l'agent hydrophobe est l'hexadécane.

49. Procédé selon l'une des revendications 42 et 43,
selon lequel
l'agent de stabilisation est un agent hydrophile.

50. Film ou membrane de structure particulaire obtenu par dépôt de nanoparticules ayant des points de reconnaissance spécifiques de molécules qui permettent une interaction spécifique entre des nanoparticules et des molécules cibles selon l'une des revendications 1 à 32 à partir de latex.

51. Film ou membrane selon la revendication 50,
dans lequel
les latex sont des microgels selon la revendication 33.

52. Film ou membrane selon l'une des revendications 50 et 51,
ayant une épaisseur de 10 nm à 1000 µm.

53. Film ou membrane selon la revendication 52 ayant une épaisseur de 10 nm à 500 µm.

54. Film ou membrane selon l'une des revendications 50 à 53, porté sur un substrat.

55. Film ou membrane selon l'une des revendications 50 à 53,
dans lequel
le substrat est une surface de détection, un stent, une membrane de filtration, un module de fibres creuses, un métal, un semi-métal, un oxyde métallique, du verre, du papier, un matériau synthétique, du nylon, de l'oxyde de polyphényle, des fibres entrelacées ou feutrées, des céramiques microporeuses, du graphite, de l'acier inoxydable, une membrane, un filtre, une feuille ou similaire.

56. Film ou membrane selon la revendication 55,
dans lequel
le semi-métal est du silicium.

57. Film ou membrane selon la revendication 55,
dans lequel
le matériau synthétique est du polyester, du polyarylsulfone, du polyéther sulfone, du polyphényle sulfone, du polyvinyle sulfone, du polystyrène, du polycarbonate, du polyéthylène, du polypropylène, du polyacétylène, de la polyvinylpiridine, du fluorure de polyvinylidène, du chlorure de polyvinyle, de la polyvinylepyrolidone, du polytétrafluoréthylène ou un de leur dérivé.

58. Film ou membrane selon l'une quelconque des revendications 50 à 57,
dans lequel
le film ou la membrane est mécaniquement stable et/ou perméable et/ ou susceptible d'être régénéré.

59. Procédé de préparation d'un film ou d'une membrane selon l'une quelconque des revendications 50 à 58,
selon lequel
on soumet au moins un latex selon la revendication 32 ou 33 à au moins une filtration et à un séchage et on obtient un film ou membrane mono ou multicouche(s) particulaire.
